(19) Europäisches Patentamt
European Patent Office
Office européen des brevets

(11) EP 1 384 713 B1

(12) **EUROPEAN PATENT SPECIFICATION**

(45) Date of publication and mention of the grant of the patent:
**15.10.2008 Bulletin 2008/42**

(21) Application number: **03076211.6**

(22) Date of filing: **21.12.1999**

(51) Int Cl.:
*C07D 223/08* (2006.01) *C07D 405/12* (2006.01)
*C07D 409/12* (2006.01) *C07D 401/12* (2006.01)
*C07D 401/14* (2006.01) *C07D 405/14* (2006.01)
*C07D 409/14* (2006.01) *C07D 471/04* (2006.01)
*C07D 417/14* (2006.01) *C07D 495/04* (2006.01)
*C07D 413/14* (2006.01) *C07D 403/12* (2006.01)
*A61K 31/55* (2006.01)

(54) **4-amino-azepan-3-one derivatives as protease inhibitors**

4-Amino-azepan-3-on-Derivate als Protease-Inhibitoren

Derives de 4-amino-azepan-3-one comme inhibiteurs de protease

(84) Designated Contracting States:
**AT BE CH CY DE DK ES FI FR GB GR IE IT LI LU MC NL PT SE**
Designated Extension States:
**RO SI**

(30) Priority: **23.12.1998 US 113636 P**
**10.11.1999 US 164581 P**

(43) Date of publication of application:
**28.01.2004 Bulletin 2004/05**

(62) Document number(s) of the earlier application(s) in accordance with Art. 76 EPC:
**99963112.0 / 1 158 986**

(73) Proprietor: **SMITHKLINE BEECHAM CORPORATION**
**Philadelphia,**
**Pennsylvania 19101-7929 (US)**

(72) Inventors:
- **Marquis, Robert Wells Jr.**
**Wayne,**
**PA 19087 (US)**
- **Ru, Yu**
**Havertown,**
**PA 19083 (US)**
- **Veber, Daniel Frank**
**Ambler, PA 19002 (US)**
- **Cummings, Maxwell David**
**Strafford, PA 19087 (US)**
- **Thompson, Scott Kevin**
**Phoenixville, PA 19460 (US)**
- **Yamashita, Dennis**
**Wayne, PA 19087 (US)**

(74) Representative: **Thompson, Clive Beresford et al**
**GlaxoSmithKline**
**Corporate Intellectual Property (CN9.25.1)**
**980 Great West Road**
**Brentford, Middlesex TW8 9GS (GB)**

(56) References cited:
**EP-A- 0 808 839** **WO-A-98/05336**

Remarks:
The file contains technical information submitted after the application was filed and not included in this specification

EP 1 384 713 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

Printed by Jouve, 75001 PARIS (FR)

## Description

### FIELD OF THE INVENTION

This invention relates in general to 4-amino-azepan-3-one protease inhibitors, particularly such inhibitors of cysteine and serine proteases, more particularly compounds which inhibit cysteine proteases, even more particularly compounds which inhibit cysteine proteases of the papain superfamily, yet more particularly compounds which inhibit cysteine proteases of the cathepsin family, most particularly compounds which inhibit cathepsin K. Such compounds are particularly useful for treating diseases in which cysteine proteases are implicated, especially diseases of excessive bone or cartilage loss, e.g., osteoporosis, periodontitis, and arthritis.

### BACKGROUND OF THE INVENTION

Cathepsins are a family of enzymes which are part of the papain superfamily of cysteine proteases. Cathepsins B, H, L, N and S have been described in the literature. Recently, cathepsin K polypeptide and the cDNA encoding such polypeptide were disclosed in U.S. Patent No. 5,501,969 (called cathepsin O therein). Cathepsin K has been recently expressed, purified, and characterized. Bossard, M. J., et al., (1996) J. Biol. Chem. 271, 12517-12524; Drake, F.H., et al., (1996) J. Biol. Chem. 271, 12511-12516; Bromme, D., et al., (1996) J. Biol. Chem. 271, 2126-2132.

Cathepsin **K** has been variously denoted as cathepsin O or cathepsin 02 in the literature. The designation cathepsin **K** is considered to be the more appropriate one.

Cathepsins function in the normal physiological process of protein degradation in animals, including humans, e.g., in the degradation of connective tissue. However, elevated levels of these enzymes in the body can result in pathological conditions leading to disease. Thus, cathepsins have been implicated as causative agents in various disease states, including but not limited to, infections by pneumocystis carinii, trypsanoma cruzi, trypsanoma brucei brucei, and Crithidia fusiculata; as well as in schistosomiasis, malaria, tumor metastasis, metachromatic leukodystrophy, muscular dystrophy, amytrophy, and the like. *See* International Publication Number WO 94/04172, published on March 3, 1994, and references cited therein. *See also* European Patent Application EP 0 603 873 A1, and references cited therein. Two bacterial cysteine proteases from P. gingivallis, called gingipains, have been implicated in the pathogenesis of gingivitis. Potempa, J., et al. (1994) Perspectives in Drug Discovery and Design, 2, 445-458.

Cathepsin K is believed to play a causative role in diseases of excessive bone or cartilage loss. Bone is composed of a protein matrix in which spindle- or plate-shaped crystals of hydroxyapatite are incorporated. Type I collagen represents the major structural protein of bone comprising approximately 90% of the protein matrix. The remaining 10% of matrix is composed of a number of non-collagenous proteins, including osteocalcin, proteoglycans, osteopontin, osteonectin, thrombospondin, fibronectin, and bone sialoprotein. Skeletal bone undergoes remodelling at discrete foci throughout life. These foci, or remodelling units, undergo a cycle consisting of a bone resorption phase followed by a phase of bone replacement.

Bone resorption is carried out by osteoclasts, which are multinuclear cells of hematopoietic lineage. The osteoclasts adhere to the bone surface and form a tight sealing zone, followed by extensive membrane ruffling on their apical (i.e., resorbing) surface. This creates an enclosed extracellular compartment on the bone surface that is acidified by proton pumps in the ruffled membrane, and into which the osteoclast secretes proteolytic enzymes. The low pH of the compartment dissolves hydroxyapatite crystals at the bone surface, while the proteolytic enzymes digest the protein matrix. In this way, a resorption lacuna, or pit, is formed. At the end of this phase of the cycle, osteoblasts lay down a new protein matrix that is subsequently mineralized. In several disease states, such as osteoporosis and Paget's disease, the normal balance between bone resorption and formation is disrupted, and there is a net loss of bone at each cycle. Ultimately, this leads to weakening of the bone and may result in increased fracture risk with minimal trauma.

Several published studies have demonstrated that inhibitors of cysteine proteases are effective at inhibiting osteoclast-mediated bone resorption, and indicate an essential role for a cysteine proteases in bone resorption. For Example, Delaisse, et al., Biochem. J., 1980, 192, 365, disclose a series of protease inhibitors in a mouse bone organ culture system and suggest that inhibitors of cysteine proteases (e.g., leupeptin, Z-Phe-Ala-$CHN_2$) prevent bone resorption, while serine protease inhibitors were ineffective. Delaisse, et al., Biochem. Biophys. Res. Commun., 1984, 125, 441, disclose that E-64 and leupeptin are also effective at preventing bone resorption *in vivo,* as measured by acute changes in serum calcium in rats on calcium deficient diets. Lerner, et al., J. Bone Min. Res., 1992, 7, 433, disclose that cystatin, an endogenous cysteine protease inhibitor, inhibits PTH stimulated bone resorption in mouse calvariae. Other studies, such as by Delaisse, et al., Bone, 1987, 8, 305, Hill, et al., J. Cell. Biochem., 1994, 56, 118, and Everts, et al., J. Cell. Physiol., 1992, 150, 221, also report a correlation between inhibition of cysteine protease activity and bone resorption. Tezuka, et al., J. Biol. Chem., 1994, 269, 1106, Inaoka, et al., Biochem. Biophys. Res. Commun., 1995, 206, 89 and Shi, et al., FEBS Lett., 1995, 357; 129 disclose that under normal conditions cathepsin K, a cysteine protease, is abundantly expressed in osteoclasts and may be the major cysteine protease present in these cells.

The abundant selective expression of cathepsin K in osteoclasts strongly suggests that this enzyme is essential for bone resorption. Thus, selective inhibition of cathepsin K may provide an effective treatment for diseases of excessive bone loss, including, but not limited to, osteoporosis, gingival diseases such as gingivitis and periodontitis, Paget's disease, hypercalcemia of malignancy, and metabolic bone disease. Cathepsin K levels have also been demonstrated to be elevated in chondroclasts of osteoarthritic synovium. Thus, selective inhibition of cathepsin K may also be useful for treating diseases of excessive cartilage or matrix degradation, including, but not limited to, osteoarthritis and rheumatoid arthritis. Metastatic neoplastic cells also typically express high levels of proteolytic enzymes that degrade the surrounding matrix. Thus, selective inhibition of cathepsin K may also be useful for treating certain neoplastic diseases.

Several cysteine protease inhibitors are known. Palmer, (1995) J. Med. Chem., 38, 3193, disclose certain vinyl sulfones which irreversibly inhibit cysteine proteases, such as the cathepsins B, L, S, 02 and cruzain. Other classes of compounds, such as aldehydes, nitriles, $\alpha$-ketocarbonyl compounds, halomethyl ketones, diazomethyl ketones, (acyloxy) methyl ketones, ketomethylsulfonium salts and epoxy succinyl compounds have also been reported to inhibit cysteine proteases. *See* Palmer, *id*, and references cited therein.

U.S. Patent No. 4,518,528 discloses peptidyl fluoromethyl ketones as irreversible inhibitors of cysteine protease. Published International Patent Application No. WO 94/04172, and European Patent Application Nos. EP 0 525 420 A1, EP 0 603 873 A1, and EP 0 611 756 A2 describe alkoxymethyl and mercaptomethyl ketones which inhibit the cysteine proteases cathepsins B, H and L. International Patent Application No. PCT/US94/08868 and and European Patent Application No. EP 0 623 592 A1 describe alkoxymethyl and mercaptomethyl ketones which inhibit the cysteine protease IL-1$\beta$convertase. Alkoxymethyl and mercaptomethyl ketones have also been described as inhibitors of the serine protease kininogenase (International Patent Application No. PCT/GB91/01479).

Azapeptides which are designed to deliver the azaamino acid to the active site of serine proteases, and which possess a good leaving group, are disclosed by Elmore et al., Biochem. J., 1968, 107, 103, Garker et al., Biochem. J., 1974, 139, 555, Gray et al., Tetrahedron, 1977, 33, 837, Gupton et al., J. Biol. Chem., 1984, 259, 4279, Powers et al., J. Biol. Chem., 1984, 259, 4288, and are known to inhibit serine proteases. In addition, J. Med. Chem., 1992, 35, 4279, discloses certain azapeptide esters as cysteine protease inhibitors.

Antipain and leupeptin are described as reversible inhibitors of cysteine protease in McConnell et al., J. Med. Chem., 33, 86; and also have been disclosed as inhibitors of serine protease in Umezawa et al., 45 Meth. Enzymol. 678. E64 and its synthetic analogs are also well-known cysteine protease inhibitors (Barrett, Biochem. J., 201, 189, and Grinde, Biochem. Biophys. Acta, , 701, 328).

1,3-diamido-propanones have been described as analgesic agents in U.S. Patent Nos.4,749,792 and 4,638,010. Thus, a structurally diverse variety of protease inhibitors have been identified. However, these known inhibitors are not considered suitable for use as therapeutic agents in animals, especially humans, because they suffer from various shortcomings. These shortcomings include lack of selectivity, cytotoxicity, poor solubility, and overly rapid plasma clearance. A need therefore exists for methods of treating diseases caused by pathological levels of proteases, particularly cysteine proteases, more particularly cathepsins, most particularly cathepsin K, and for novel inhibitor compounds useful in such methods.

International Patent Application, Publication Number 98/05336 does disclose certain pyrrolidine, piperidine and morpholine derivatives being inhibitors of cysteine proteases, particularly cathepsin K, useful in the treatment of diseases in which inhibition of bone mass is a factor.

We have now discovered a novel class of 4-amino-azepan-3-one compounds which are protease inhibitors, most particularly of cathepsin K.

## SUMMARY OF THE INVENTION

An object of the present invention is to provide 4-amino-azepan-3-one carbonyl protease inhibitors, particularly such inhibitors of cysteine and serine proteases, more particularly such compounds which inhibit cysteine proteases, even more particularly such compounds which inhibit cysteine proteases of the papain superfamily, yet more particularly such compounds which inhibit cysteine proteases of the cathepsin family, most particularly such compounds which inhibit cathepsin K, and which are useful for treating diseases which may be therapeutically modified by altering the activity of such proteases.

Accordingly, in the first aspect, this invention provides a compound according to Formula I.

In another aspect, this invention provides a pharmaceutical composition comprising a compound according to Formula I and a pharmaceutically acceptable carrier, diluent or excipient.

In still another aspect, this invention provides a compound of Formula I for use in treating diseases in which the disease pathology may be therapeutically modified by inhibiting proteases, particularly cysteine and serine proteases.

In a particular aspect, the compounds of this invention are especially useful for treating diseases characterized by bone loss, such as osteoporosis and gingival diseases, such as gingivitis and periodontitis, or by excessive cartilage or matrix degradation, such as osteoarthritis and rheumatoid arthritis.

## DETAILED DESCRIPTION OF THE INVENTION

The present invention provides compounds of Formula I:

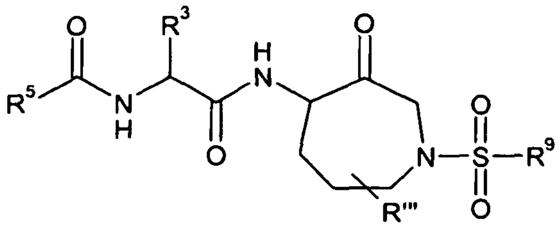

(I)

wherein,

$R^5$ is selected from the group consisting of 3-methyl-benzofuranyl-2-yl, thieno[3,2,*b*]thiophen-2-yl, 5-methoxybenzofuran-2-yl, quinoxalin-2-yl and quinolin-2-yl;
R"' is hydrogen
$R^3$ is isobutyl;
R9 is selected from the group consisting of pyridine-2-yl and 1-oxy-pyridin-2-yl; and pharmaceutical salts, hydrates and solvates thereof.

In addition, purely for information, reference is also made herein to compounds of Formula A:

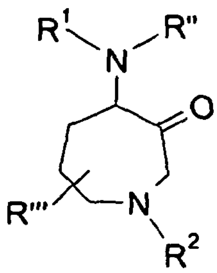

A

wherein:

$R^1$ is selected from the group consisting of:

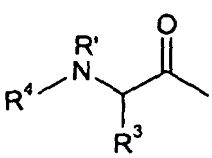
;
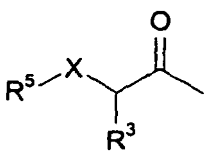
. and
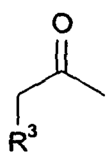
;

$R^2$ is selected from the group consisting of: H, $C_{1-6}$alkyl, $C_{3-6}$cycloalkyl-$C_{0-6}$alkyl, Ar-$C_{0-6}$alkyl, Het-$C_{0-6}$alkyl, $R^9C(O)$-, $R^9C(S)$-, $R^9SO_2$-, $R^9OC(O)$-, $R^9R^{11}NC(O)$-, $R^9R^{11}NC(S)$-, $R^9(R^{11})NSO_2$-

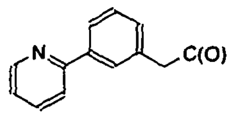,
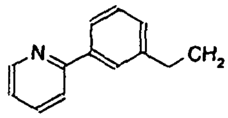

and

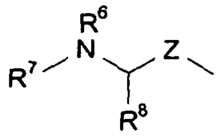
;

$R^3$ is selected from the group consisting of: H, $C_{1-6}$alkyl, $C_{2-6}$alkenyl, $C_{2-6}$alkynyl, Het$C_{0-6}$alkyl and Ar$C_{0-6}$alkyl;
$R^3$ and R’ may be connected to form a pyrrolidine (204), piperidine or morpholine ring;
$R^4$ is selected from the group consisting of: H, $C_{1-6}$alkyl, $C_{3-6}$cycloalkyl-$C_{0-6}$alkyl, Ar-$C_{0-6}$alkyl, Het-$C_{0-6}$alkyl, $R^5$C(O)-, $R^5$C(S)-, $R^5SO_2$-, $R^5$OC(O)-, $R^5R^{13}$NC(O)-, and $R^5R^{13}$NC(S)-;
$R^5$ is selected from the group consisting of: H, $C_{1-6}$alkyl, $C_{2-6}$alkenyl, $C_{2-6}$alkynyl, $C_{3-6}$cycloalkyl-$C_{0-6}$alkyl, Ar-$C_{0-6}$alkyl and Het-$C_{0-6}$alkyl;
$R^6$ is selected from the group consisting of: H, $C_{1-6}$(alkyl, Ar-$C_{0-6}$alkyl, and Het-$C_{0-6}$alkyl;
$R^7$ is selected from the group consisting of: H, $C_{1-6}$alkyl, $C_{3-6}$cycloalkyl-$C_{0-6}$alkyl, Ar-$C_{0-6}$alkyl, Het-$C_{0-6}$alkyl, $R^{10}$C(O)-, $R^{10}$C(S)-, $R^{10}SO_2$-, $R^{10}$OC(O)-, $R^{10}R^{14}$NC(O)-, and $R^{10}R^{14}$NC(S)-;
$R^8$ is selected from the group consisting of H, $C_{1-6}$alkyl, $C_{2-6}$alkenyl, $C_{2-6}$alkynyl, Het$C_{0-6}$alkyl and Ar$C_{0-6}$alkyl;
$R^9$ is selected from the group consisting of: $C_{1-6}$alkyl, $C_{3-6}$cycloalkyl-$C_{0-6}$alkyl, Ar-$C_{0-6}$alkyl and Het-$C_{0-6}$alkyl;
$R^{10}$ is selected from the group consisting of: $C_{1-6}$alkyl, $C_{3-6}$cycloalkyl-$C_{0-6}$alkyl, Ar-$C_{0-6}$alkyl and Het-$C_{0-6}$alkyl;
$R^{11}$ is selected from the group consisting of: H, $C_{1-6}$alkyl, Ar-$C_{0-6}$alkyl, and Het-$C_{0-6}$alkyl;
$R^{12}$ is selected from the group consisting of: H, $C_{1-6}$alkyl, Ar-$C_{0-6}$alkyl, and Het-$C_{0-6}$alkyl;
$R^{13}$ is selected from the group consisting of: H, $C_{1-6}$alkyl, Ar-$C_{0-6}$alkyl, and Het-$C_{0-6}$alkyl;
$R^{14}$ is selected from the group consisting of: H, $C_{1-6}$alkyl, Ar-$C_{0-6}$alkyl, and Het-$C_{0-6}$alkyl;
R’ is selected from the group consisting of: H, $C_{1-6}$alkyl, Ar-$C_{0-6}$alkyl, and Het-$C_{0-6}$alkyl;
R" is selected from the group consisting of: H, $C_{1-6}$alkyl, Ar-$C_{0-6}$alkyl, or Het-$C_{0-6}$alkyl;
R"’ is selected from the group consisting of: H, $C_{1-6}$alkyl, $C_{3-6}$cycloalkyl-$C_{0-6}$alkyl, Ar-$C_{0-6}$alkyl, and Het-$C_{0-6}$alkyl;
X is selected from the group consisting of: $CH_2$, S, and O;
Z is selected from the group consisting of: C(O) and $CH_2$;

and pharmaceutically acceptable salts, hydrates and solvates thereof.

Compounds of Formula I selected from the following group are particularly preferred embodiments of the present invention:

| Example No. | Chemical Name |
|---|---|
| 1 | Quinoline-2-carboxylic acid {(S)-3-methyl-1-[3-oxo-1-(pyridine-2-sulfonyl)-azepan-4-ylcarbamoyl]-butyl} amide |
| 2 | 5-Methoxybenzofuran-2-carboxylic acid {(S)-3-methyl-1-[3-oxo-1-(pyridine-2-sulfonyl)-azepan-4-ylcarbamoyl]-butyl} amide |
| 3 | 5-Methoxy-benzofuran-2-carboxylic acid {(S)-3-methyl-1-[3-oxo-1-(1-oxy-pyridine-2-sulfonyl)-azepan-4-ylcarbamoyl]-butyl}amide |
| 4 | Thieno[3,2-b]thiophene-2-carboxylic acid {(S)-3-methyl-1-[3-oxo-1-(1-oxy-pyridine-2-sulfonyl)-azepan-4-ylcarbamoyl]-butyl} amide |
| 5 | Quinoxaline-2-carboxylic acid {(S)-3-methyl-1-[3-oxo-1-(1-oxy-pyridine-2-sulfonyl)-azepan-4-ylcarbamoyl]-butyl}amide |
| 6 | 3-Methyl-benzofuran-2-carboxylic acid {(S)-3-methyl-1-[3-oxo-1-(1-oxy-pyridine-2-sulfonyl)-azepan-4-ylcarbamoyl]-butyl} amide |
| 7 | 3-Methyl-benzofuran-2-carboxylic acid {(S)-3-methyl-1-[3-oxo-1-(pyridine-2-sulfonyl)-azepan-4-ylcarbamoyl]-butyl} amide |

(continued)

| Example No. | Chemical Name |
|---|---|
| 8 | Thieno[3,2-b]thiophene-2-carboxylic acid {(S)-3-methyl-1-[3-oxo-1-(pyridine-2-sulfonyl)-azepan-4-ylcarbamoyl]-butyl} amide |

Specific representative compounds of the present invention are set forth in Examples 1 to 8.

Compared to the corresponding 5 and 6 membered ring compounds, the 7 membered ring compounds of the present invention are configurationally more stable at the carbon center alpha to the ketone.

The present invention includes deuterated analogs of the inventive compounds. A demonstration Example of such a deuterated compound is set forth in (Demonstration) Example 9. A representative synthetic route for the deuterated compounds of the present invention is set forth in Scheme 4, below. The deuterated compounds of the present invention exhibit superior chiral stability compared to the protonated isomer.

## Definitions

The present invention includes all pharmaceutical salts, hydrates and solvates of the compounds of this invention. If a chiral center or another form of an isomeric center is present in a compound of the present invention, all forms of such isomer or isomers, including enantiomers and diastereomers, are intended to be covered herein. Inventive compounds containing a chiral center may be used as a racemic mixture, an enantiomerically enriched mixture, or the racemic mixture may be separated using well-known techniques and an individual enantiomer may be used alone. In cases in which compounds have unsaturated carbon-carbon double bonds, both the cis (Z) and trans (E) isomers are within the scope of this invention. In cases wherein compounds may exist in tautomeric forms, such as keto-enol tautomers, each tautomeric form is contemplated as being included within this invention whether existing in equilibrium or predominantly in one form.

The meaning of any substituent at any one occurrence in Formula I or any subformula thereof is independent of its meaning, or any other substituent's meaning, at any other occurrence, unless specified otherwise.

Abbreviations and symbols commonly used in the peptide and chemical arts are used herein to describe the compounds of the present invention. In general, the amino acid abbreviations follow the IUPAC-IUB Joint Commission on Biochemical Nomenclature as described in Eur. J. Biochem., 158, 9 (1984).

"Proteases" are enzymes that catalyze the cleavage of amide bonds of peptides and proteins by nucleophilic substitution at the amide bond, ultimately resulting in hydrolysis. Such proteases include: cysteine proteases, serine proteases, aspartic proteases, and metalloproteases. The compounds of the present invention are capable of binding more strongly to the enzyme than the substrate and in general are not subject to cleavage after enzyme catalyzed attack by the nucleophile. They therefore competitively prevent proteases from recognizing and hydrolyzing natural substrates and thereby act as inhibitors.

The term "amino acid" as used herein refers to the D- or L- isomers of alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine and valine.

"$C_{1-6}$alkyl" as applied herein is meant to include substituted and unsubstituted methyl, ethyl, n-propyl, isopropyl, n-butyl, isobutyl and t-butyl, pentyl, n-pentyl, isopentyl, neopentyl and hexyl and the simple aliphatic isomers thereof. $C_{1-6}$alky may be optionally substituted by a moiety selected from the group consisting of: $OR^{12}$, $C(O)R^{12}$, $SR^{12}$, $S(O)R^{12}$, $NR^{12}_2$, $R^{12}NC(O)OR^5$, $CO_2R^{12}$, $CO_2NR^{12}_2$, $N(C{=}NH)NH_2$, Het, $C_{3-6}$cycloalkyl, and Ar; where $R^5$ is selected from the group consisting of: H, $C_{1-6}$alkyl, $C_{2-6}$alkenyl, $C_{2-6}$alkynyl, $C_{3-6}$cycloalkyl-$C_{0-6}$alkyl, Ar-$C_{0-6}$alkyl and Het-$C_{0-6}$alkyl; and $R^{12}$ is selected from the group consisting of: H, $C_{1-6}$alkyl, Ar-$C_{0-6}$alkyl, and Het-$C_{0-6}$alkyl;

"$C_{3-6}$cycloalkyl" as applied herein is meant to include substituted and unsubstituted cyclopropane, cyclobutane, cyclopentane and cyclohexane.

"$C_{2-6}$ alkenyl" as applied herein means an alkyl group of 2 to 6 carbons wherein a carbon-carbon single bond is replaced by a carbon-carbon double bond. $C_{2-6}$alkenyl includes ethylene, 1-propene, 2-propene, 1-butene, 2-butene, isobutene and the several isomeric pentenes and hexenes. Both cis and trans isomers are included.

"$C_{2-6}$alkynyl" means an alkyl group of 2 to 6 carbons wherein one carbon-carbon single bond is replaced by a carbon-carbon triple bond. $C_{2-6}$ alkynyl includes acetylene, 1-propyne, 2-propyne, 1-butyne, 2-butyne, 3-butyne and the simple isomers of pentyne and hexyne.

"Halogen" means F, Cl, Br, and I.

"Ar" or "aryl" means phenyl or naphthyl, optionally substituted by one or more of Ph-$C_{0-6}$alkyl; Het-$C_{0-6}$alkyl; $C_{1-6}$alkoxy; Ph-$C_{0-6}$alkoxy; Het-$C_{0-6}$alkoxy; OH, $(CH_2)_{1-6}NR^{15}R^{16}$; $O(CH_2)_{1-6}NR^{15}R^{16}$; $C_{1-6}$alkyl, $OR^{17}$, $N(R^{17})_2$, $SR^{17}$, $CF_3$, $NO_2$, CN, $CO_2R^{17}$, $CON(R^{17})$, F, Cl, Br or I; where $R^{15}$ and $R^{16}$ are H, $C_{1-6}$alkyl, Ph-$C_{0-6}$alkyl, naphthyl-$C_{0-6}$alkyl or Het-$C_{0-6}$alkyl; and $R^{17}$ is phenyl, naphthyl, or $C_{1-6}$alkyl.

As used herein "Het" or "heterocyclic" represents a stable 5- to 7-membered monocyclic, a stable 7- to 10-membered bicyclic, or a stable 11- to 18-membered tricyclic heterocyclic ring which is either saturated or unsaturated, and which consists of carbon atoms and from one to three heteroatoms selected from the group consisting of N, O and S, and wherein the nitrogen and sulfur heteroatoms may optionally be oxidized, and the nitrogen heteroatom may optionally be quaternized, and including any bicyclic group in which any of the above-defined heterocyclic rings is fused to a benzene ring. The heterocyclic ring may be attached at any heteroatom or carbon atom which results in the creation of a stable structure, and may optionally be substituted with one or two moieties selected from $C_{0-6}$Ar, $C_{1-6}$alkyl, $OR^{17}$, $N(R^{17})_2$, $SR^{17}$, $CF_3$, $NO_2$, CN, $CO_2R^{17}$, $CON(R^{17})$, F, Cl, Br and I, where $R^{17}$ is phenyl, naphthyl, or $C_{1-6}$alkyl. Examples of such heterocycles include piperidinyl, piperazinyl, 2-oxopiperazinyl, 2-oxopiperidinyl, 2-oxopyrrolodinyl, 2-oxoazepinyl, azepinyl, pyrrolyl, 4-piperidonyl, pyrrolidinyl, pyrazolyl, pyrazolidinyl, imidazolyl, pyridinyl, 1-oxo-pyridinyl, pyrazinyl, oxazolidinyl, oxazolinyl, oxazolyl, isoxazolyl, morpholinyl, thiazolidinyl, thiazolinyl, thiazolyl, quinuclidinyl, indolyl, quinolinyl, quinoxalinyl, isoquinolinyl, benzimidazolyl, benzopyranyl, benzoxazolyl, furanyl, benzofuranyl, thiophenyl, benzo[b]thiophenyl, thieno[3,2-b]thiophenyl, benzo[1,3]dioxolyl, 1,8 naphthyridinyl, pyranyl, tetrahydrofuranyl, tetrahydropyranyl, thienyl, benzoxazolyl, thiamorpholinyl sulfoxide, thiamorpholinyl sulfone, and oxadiazolyl, as well as triazolyl, thiadiazolyl, oxadiazolyl, isothiazolyl, imidazolyl, pyridazinyl, pyrimidinyl, triazinyl and tetrazinyl which are available by routine chemical synthesis and are stable. The term heteroatom as applied herein refers to oxygen, nitrogen and sulfur.

Here and throughout this application the term $C_0$ denotes the absence of the substituent group immediately following; for instance, in the moiety $ArC_{0-6}$alkyl, when C is 0, the substituent is Ar, e.g., phenyl. Conversely, when the moiety $ArC_{0-6}$alkyl is identified as a specific aromatic group, e.g., phenyl, it is understood that the value of C is 0.

Certain radical groups are abbreviated herein. t-Bu refers to the tertiary butyl radical, Boc refers to the t-butyloxycarbonyl radical, Fmoc refers to the fluorenylmethoxycarbonyl radical, Ph refers to the phenyl radical, Cbz refers to the benzyloxycarbonyl radical.

Certain reagents are abbreviated herein. m-CPBA refers to 3-chloroperoxybenzoic acid, EDC refers to N-ethyl-N'(dimethylaminopropyl)-carbodiimide, DMF refers to dimethyl formamide, DMSO refers to dimethyl sulfoxide, TEA refers to triethylamine, TFA refers to trifluoroacetic acid, and THF refers to tetrahydrofuran.

**Methods of Preparation**

Compounds of the general Formula A and as appropriate the compounds of the invention of Formula I may be prepared in a fashion analogous to that outlined in Schemes 1, 2 and 3. Alkylation of *tert*-butyl N-allylcarbamate (**1**) with a base such as sodium hydride and 5-bromo-1-pentene provides the diene **2**. Treatment of **2** with either 2,6-diisopropylphenylimido neophylidene molybenum bis(tert-butoxide) or bis(tricyclohexylphosphine)benzylidine ruthenium (IV) dichloride olefin metathesis catalysts developed by Grubbs provides the azepine **3**. Epoxidation of **3** with standard oxidizing agents common to the art such as *m*-CPBA provide the epoxide **4**. Nucleophilic epoxide ring opening may be effected with a reagent such as sodium azide to provide the azido alcohol (not shown) which may be reduced to the amino alcohol **5** under conditions common to the art such as 1,3-propanedithiol and triethylamine in methanol or with hydrogen gas in the presence of a catalyst such as palladium on carbon. Acylation of **5** with an acid such as Cbz-leucine in the presence of a coupling agent such as EDC followed by removal of the BOC protecting group under acidic conditions provides the amine salt **6**. Coupling of **6** with Cbz-leucine may be effected with a coupling agent such as EDC to provide the intermediate alcohol (not shown) which was oxidized with an oxidant such as pyridine sulfur trioxide complex in DMSO and triethylamine to provide the ketone 7.

## Scheme 1

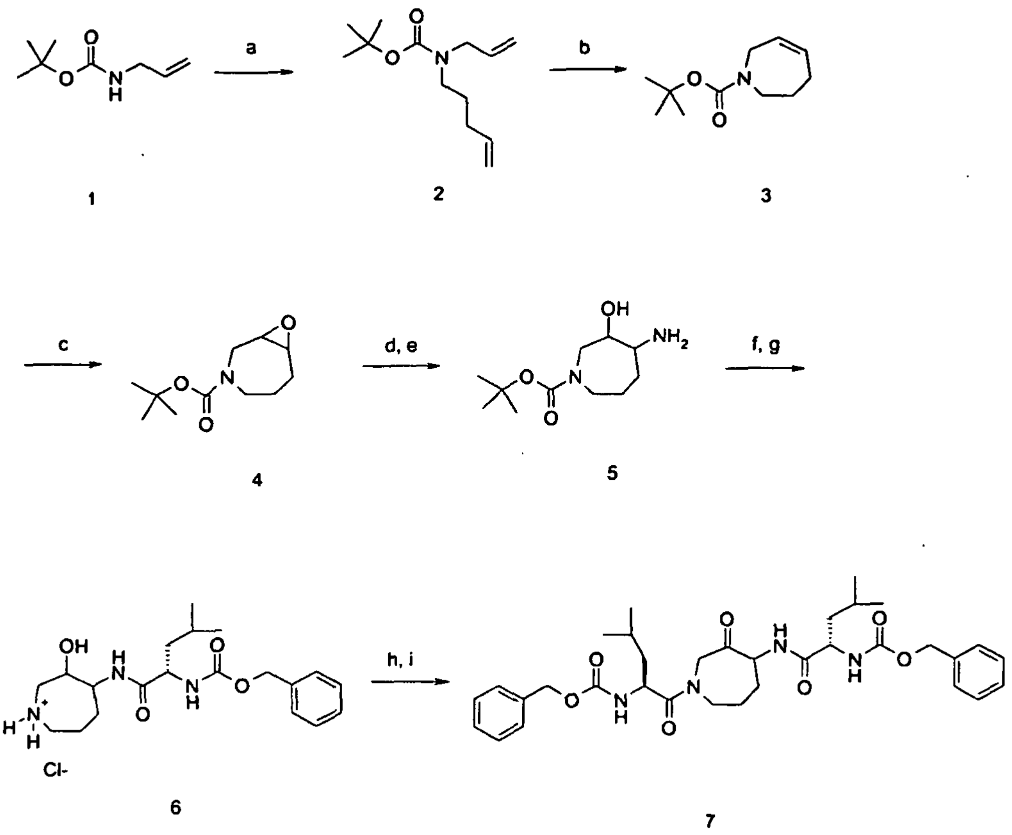


1 2 3 4 5 6 7

**Reagents and conditions:** a.) NaH, 5-bromo-1-pentene, DMF; b.) 2,6-diisopropylphenylimido neophylidene molybenum bis(tert-butoxide) or bis(tricyclohexylphosphine)benzylidine ruthenium (IV) dichloride catalyst, toluene c.) *m*-CPBA, $CH_2Cl_2$; d.) $NaN_3$, $CH_3OH$, $H_2O$, $NH_4Cl$; e.) 10% Pd/C, $H_2$, f.) Cbz-leucine, EDC, $CH_2Cl_2$; g.) HCl, EtOAc; h.) Cbz-leucine, EDC, $CH_2Cl_2$; i.) pyridine sulfur trioxide complex, DMSO, TEA.

Compounds of the general Formula A and as appropriate the compounds of the invention of Formula I wherein $R^1$ and $R^2$ are amides may be prepared in the general fashion outlined in Scheme 2. Alkylation ofN-Cbz allyl amine (**8**) with a base such as sodium hydride and 5-bromo-1-pentene provides the diene **9.** Treatment of **9** with bis(tricyclohexylphosphine)benzylidine ruthenium(IV)dichloride olefin metathesis catalyst developed by Grubbs provides the azepine 10. Epoxidation of **10** with standard oxidizing agents common to the art such as *m*-CPBA provide the epoxide **11.** Nucleophilic epoxide ring opening may be effected with a reagent such as sodium azide to provide the azido alcohol (not shown) which may be reduced to the amino alcohol **12** with a reducing agent such as propanedithiol in the presence of triethylamine. Acylation of **12** with N-Boc-leucine and a coupling agent such as EDC followed by removal of the Cbz protecting group under hydrogenolysis conditions provides the amine **13.** Coupling of **13** with a carboxylic acid was effected with a coupling agent such as EDC followed by removal of the acid labile N-Boc protecting group with an acid such as HCl or TFA provides intermediate **14**. Acylation of **14** may be effected with a carboxylic acid in the presence of a coupling agent common to the art such as EDC to give the intermediate alcohol (not shown) which is oxidized with an oxidant such as pyridine sulfur trioxide complex in DMSO and triethylamine to provide the ketone **15**.

## Scheme 2

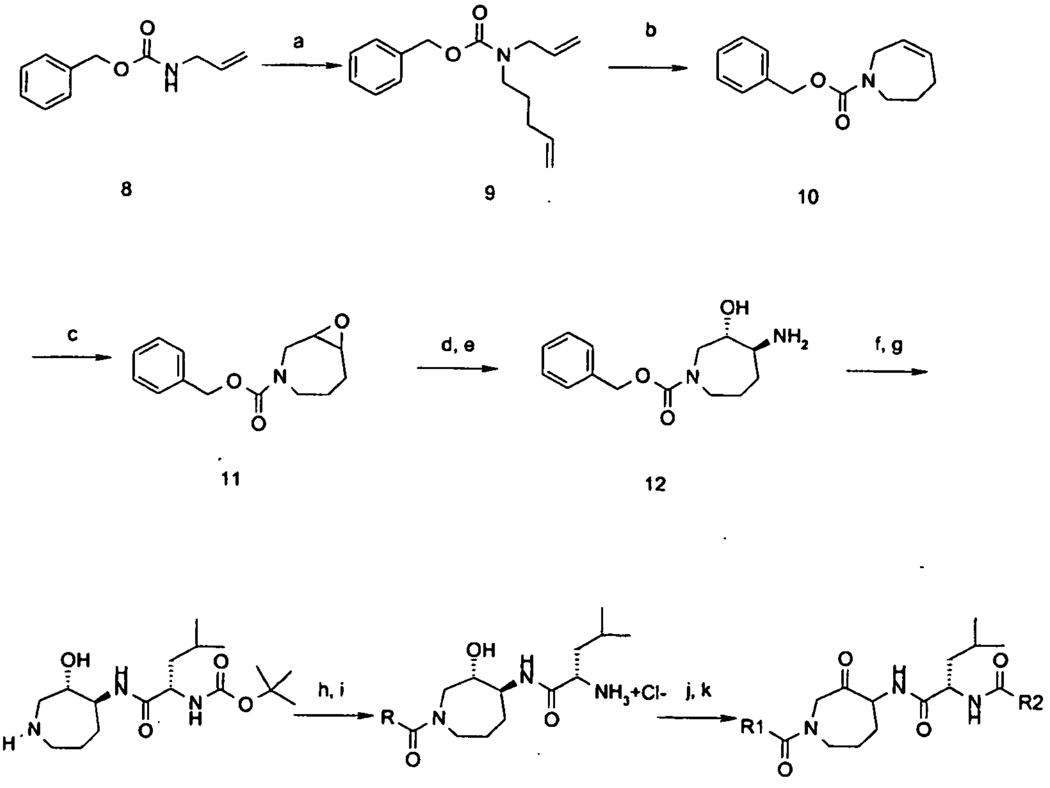


8 9 10 11 12 13 14 15

**Reagents and conditions:** a.) NaH, 5-bromo-1-pentene, DMF; b.) bis(tricyclohexylphosphine)benzylidine ruthenium (IV) dichloride catalyst, $CH_2Cl_2$; c.) *m*-CPBA, $CH_2Cl_2$; d.) $NaN_3$, $CH_3OH$, $H_2O$, $NH_4Cl$; e.) propanedithiol, $CH_3OH$, TEA; f.) Boc-leucine, EDC, $CH_2Cl_2$; g.) 10% Pd/C, $H_2$; h.) $R_1CO_2H$, EDC, $CH_2Cl_2$ or $R_1COCl$, $CH_2Cl_2$; i.) HCl/ EtOAc; j.) $R_2CO_2H$, EDC, $CH_2Cl_2$; k.) pyridine sulfur trioxide complex, DMSO, TEA.

Compounds of the general Formula A and as appropriate the compounds of the invention of Formula I wherein $R^2$ is an alkyl, urea or sulphonamide group and $R^1$ is an amide may be prepared in the general fashion outlined in Scheme 3. Reductive amination of **13** may be effected by treatment with an aldehyde followed by a reducing agent such as sodium triacetoxyborohydride. Subsequent deprotection of the N-Boc group under acidic conditions provides the amine salt **16**. Coupling of **16** with an acid chloride or with a carboxylic acid in the presence of a coupling agent common to the art such as EDC followed by oxidation of the intermediate alcohol (not shown) with an oxidant such as pyridine sulfur trioxide complex provides the ketone **17**. Alternatively, treatment of amine **13** with an isocyanate followed by deprotection of the N-Boc group provides the amine salt **18**. Acylation and oxidation provides the ketone **19**. Further derivatization of amine **13** may be effected by treatment with a sulphonyl chloride followed by deprotection of the N-Boc group to provide the amine salt **20**. Acylation and oxidation provides the ketone **21**.

## Scheme 3

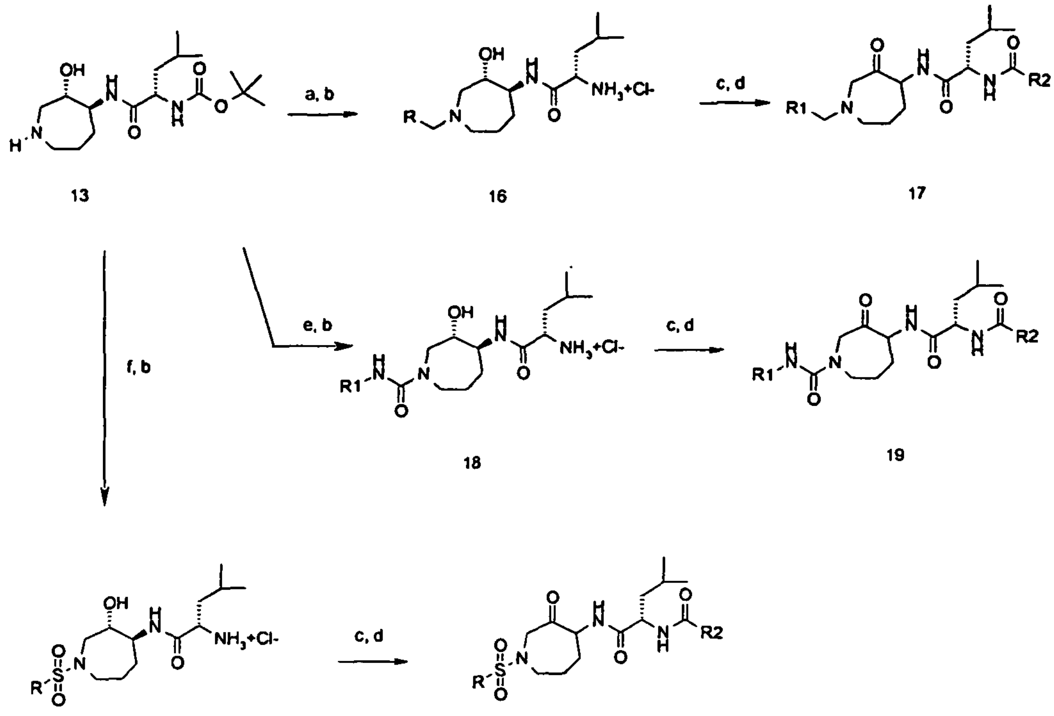


13 16 17 18 19 20 21

**Reagents and conditions:** a.) $R_1CHO$, $NaBH(OAC)_3$; b.) HCl; c.) $R_2CO_2H$, EDC, $CH_2Cl_2$; d.) pyridine sulfur trioxide complex, DMSO, TEA; e.) $R_1NCO$, base; f.) $R_1SO_2Cl$, TEA, $CH_2Cl_2$.

The deuterated compound of the (Demonstration) Example 9 may be conveniently prepared according to Scheme 4. The skilled artisan will understand from (Demonstration) Example 9 and Scheme 4 how to make any of the the deuterated compounds of the present invention.

The individual diastereomers of benzofuran-2-carboxylic acid {(S)-3-methyl-1-[(2,2',4-trideuterio)-3-oxo-1-(pyridine-2-sulfonyl)-azepan-4-ylcarbamoyl]-butyl}amide **31** and **32** may be prepared as outlined in Scheme 4. Alkylation of allyl-carbamic acid benzyl ester **22** with 5-bromo-1-pentene in the presence of a base such as sodium hydride provides the diene **23**. Treatment of diene **23** with bis(tricyclohexylphosphine)benzylidine ruthenium (IV) dichloride developed by Grubbs provides the 2,3,4,7-tetrahydro-azepine-1-carboxylic acid benzyl ester **24**. Epoxidation of azepine **24** may be effected with standard oxidizing agents common to the art such as *m*-CPBA to provide epoxide **25**. Nucleophilic epoxide ring opening of **25** may be effected with a reagent such as sodium azide to provide the azido alcohol (not shown).

## Scheme 4

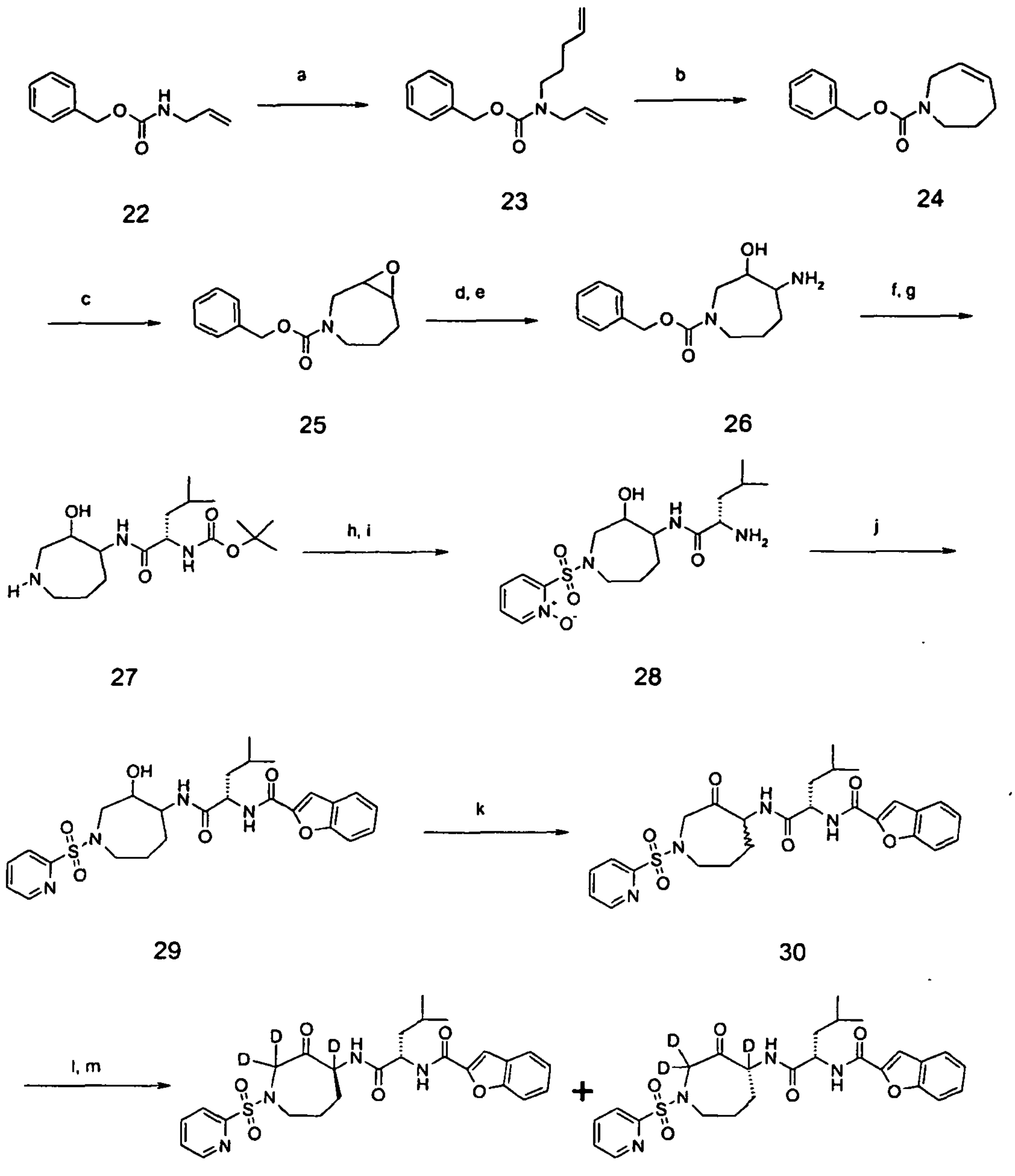


22 23 24 25 26 27 28 29 30 31 32

**Reagents and Conditions:** a.) NaH, 5-bromo-1-pentene, DMF; b.) bis(tricyclohexylphosphine)benzylidine ruthenium (IV) dichloride, $CH_2Cl_2$; c.) *m*-CPBA, $CH_2Cl_2$; d.) $NaN_3$, $CH_3OH$, $H_2O$, $NH_4Cl$; e.) 1,3-propanedithiol, TEA, methanol; f.) N-Boc-leucine, EDC, $CH_2Cl_2$; g.) 10% Pd/C, $H_2$; h.) 2-pyridinesulphonyl chloride, TEA, $CH_2Cl_2$; i.) 4 N HCl/dioxane, methanol; j.) benzofuran-2-carboxylic acid, EDC, $CH_2Cl_2$; k.) pyridine sulfur trioxide complex, DMSO, TEA; 1.) $CD_3OD$; $D_2O$ (10:1), TEA; m.) HPLC separation. The intermediate azido alcohol may be reduced to the amino alcohol **26** under conditions common to the art such as 1,3-propanedithiol and triethylamine in methanol or with triphenylphosphine in tetrahydrofuran and water. Acylation of **26** may be effected with an acid such as N-Boc-leucine in the presence of a coupling agent such as EDC. Removal of the benzyloxycarbonyl protecting group with hydrogen gas in the presence of 10% Pd/C provides the amine **27**. Treatment of the amine **27** with 2-pyridinesulphonyl chloride in the presence of triethylamine or saturated sodium bicarbonate and $CH_2Cl_2$ followed by removal of the *tert*-butoxycarbonyl protecting group under acidic conditions provides **28**. Coupling of **28** with benzofuran-2-carboxylic acid may be effected with a

coupling agent such as EDC to provide intermediate alcohol **29.** Alcohol **29** may be oxidized with an oxidant such as sulfur trioxide pyridine complex in DMSO and triethylamine to provide the ketone **30** as a mixture of diastereomers. Treatment of ketone **30** with triethylamine in $CD_3OD$:$D_2O$ at reflux provides the deuterated analog as a mixture of diastereomers which are separated by HPLC to provide the deuterated compounds **31** and **32**.

Compounds of the general formula A and as appropriate the compounds of the invention of Formula Imay also be prepared as outlined in Scheme 5. The amine of compound **12** may be protected with with di-*tert*-butyldicarbonate to provide the N-Boc derivative **33** (Scheme 2). Removal of the benzyloxycarbonyl protecting group may be effected by treatment of **33** with hydrogen gas in the presence of a catalyst such as 10% Pd/C to provide the amine **34**. Treatment of amine **34** with a sulfonyl chloride such as 2-pyridinesulfonyl chloride in the presence of a base such as N-methylmorpholine or triethylamine provides the sulfonamide derivative **35**. Removal of the *tert*-butoxycarbonyl protecting group may be effected with an acid such as hydrochloric acid to provide intermediate **36**. Coupling of **36** with an acid such as N-Boc-cyclohexylalanine in the presence of a coupling agent common to the art such as HBTU or polymer supported EDC provides the alcohol intermediate **37**. Removal of the *tert*-butoxycarbonyl protecting group under acidic conditions provides amine **38**. Coupling of **38** with an acid such as benzofuran-2-carboxylic acid in the presence of a coupling agent such as HBTU or polymer supported EDC provides alcohol **39**. Alcohol **39** may be oxidized with an oxidant common to the art such as pyridine sulfur trioxide complex in DMSO and triethylamine or the Dess-Martin periodinane to provide the ketone **40**.

Scheme 5

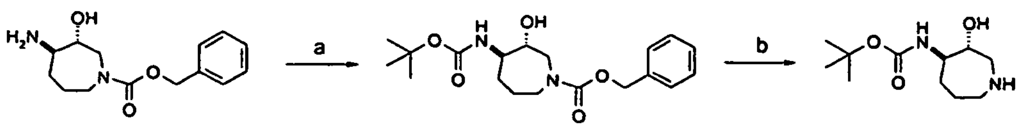


12 33 34

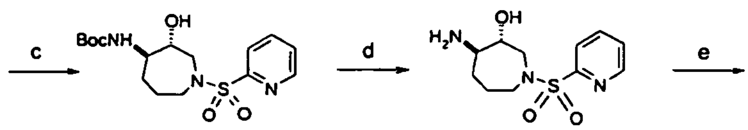


35 36

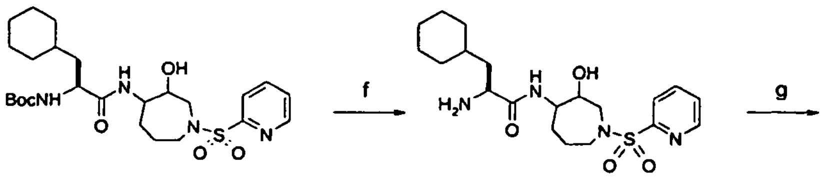


37 38

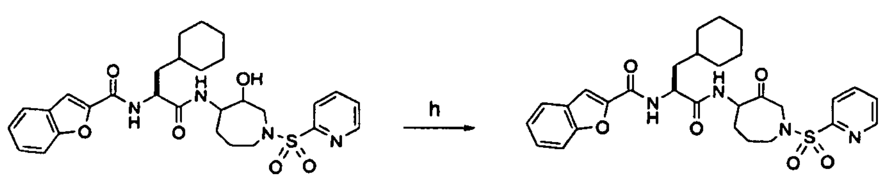


39 40

**Reagents and Conditions:** (a) Di-*tert*-butyldicarbonate, THF; (b) $H_2$, 10% Pd/C, EtOAc; (c) 2-pyridylsulfonyl chloride, TEA ; (d) HCl, EtOAc; (e) N-Boc-cylohexylalanine, P-EDC, $CH_2Cl_2$; (f) HCl, $CH_2Cl_2$; (g) benzofuran-2-carboxylic acid, P-EDC, $CH_2Cl_2$; (h) Dess-Martin periodinane, methylene chloride.

The starting materials used herein are commercially available amino acids or are prepared by routine methods well known to those of ordinary skill in the art and can be found in standard reference books, such as the COMPENDIUM

OF ORGANIC SYNTHETIC METHODS, Vol. I-VI (published by Wiley-Interscience).

Coupling methods to form amide bonds herein are generally well known to the art. The methods of peptide synthesis generally set forth by Bodansky et al., THE PRACTICE OF PEPTIDE SYNTHESIS, Springer-Verlag, Berlin, 1984; E. Gross and J. Meienhofer, THE PEPTIDES, Vol. 1, 1-284 (1979); and J.M. Stewart and J.D. Young, SOLID PHASE PEPTIDE SYNTHESIS, 2d Ed., Pierce Chemical Co., Rockford, Ill., 1984. are generally illustrative of the technique and are incorporated herein by reference.

Synthetic methods to prepare the compounds of this invention frequently employ protective groups to mask a reactive functionality or minimize unwanted side reactions. Such protective groups are described generally in Green, T.W, PROTECTIVE GROUPS IN ORGANIC SYNTHESIS, John Wiley & Sons, New York (1981). The term "amino protecting groups" generally refers to the Boc, acetyl, benzoyl, Fmoc and Cbz groups and derivatives thereof as known to the art. Methods for protection and deprotection, and replacement of an amino protecting group with another moiety are well known.

Acid addition salts of the compounds of Formula A and as appropriate the compounds of the invention of Formula I are prepared in a standard manner in a suitable solvent from the parent compound and an excess of an acid, such as hydrochloric, hydrobromic, hydrofluoric, sulfuric, phosphoric, acetic, trifluoroacetic, maleic, succinic or methanesulfonic. Certain of the compounds form inner salts or zwitterions which may be acceptable. Cationic salts are prepared by treating the parent compound with an excess of an alkaline reagent, such as a hydroxide, carbonate or alkoxide, containing the appropriate cation; or with an appropriate organic amine. Cations such as $Li^+$, $Na^+$, $K^+$, $Ca^{++}$, $Mg^{++}$ and $NH_4^+$ are specific Examples of cations present in pharmaceutically acceptable salts. Halides, sulfate, phosphate, alkanoates (such as acetate and trifluoroacetate), benzoates, and sulfonates (such as mesylate) are Examples of anions present in pharmaceutically acceptable salts.

This invention also provides a pharmaceutical composition which comprises a compound according to Formula I and a pharmaceutically acceptable carrier, diluent or excipient. Accordingly, the compounds of Formula I may be used in the manufacture of a medicament. Pharmaceutical compositions of the compounds of Formula I prepared as hereinbefore described may be formulated as solutions or lyophilized powders for parenteral administration. Powders may be reconstituted by addition of a suitable diluent or other pharmaceutically acceptable carrier prior to use. The liquid formulation may be a buffered, isotonic, aqueous solution. Examples of suitable diluents are normal isotonic saline solution, standard 5% dextrose in water or buffered sodium or ammonium acetate solution. Such formulation is especially suitable for parenteral administration, but may also be used for oral administration or contained in a metered dose inhaler or nebulizer for insufflation. It may be desirable to add excipients such as polyvinylpyrrolidone, gelatin, hydroxy cellulose, acacia, polyethylene glycol, mannitol, sodium chloride or sodium citrate.

Alternately, these compounds may be encapsulated, tableted or prepared in an emulsion or syrup for oral administration. Pharmaceutically acceptable solid or liquid carriers may be added to enhance or stabilize the composition, or to facilitate preparation of the composition. Solid carriers include starch, lactose, calcium sulfate dihydrate, terra alba, magnesium stearate or stearic acid, talc, pectin, acacia, agar or gelatin. Liquid carriers include syrup, peanut oil, olive oil, saline and water. The carrier may also include a sustained release material such as glyceryl monostearate or glyceryl distearate, alone or with a wax. The amount of solid carrier varies but, preferably, will be between about 20 mg to about 1g per dosage unit. The pharmaceutical preparations are made following the conventional techniques of pharmacy involving milling, mixing, granulating, and compressing, when necessary, for tablet forms; or milling, mixing and filling for hard gelatin capsule forms. When a liquid carrier is used, the preparation will be in the form of a syrup, elixir, emulsion or an aqueous or non-aqueous suspension. Such a liquid formulation may be administered directly p.o. or filled into a soft gelatin capsule.

For rectal administration, the compounds of this invention may also be combined with excipients such as cocoa butter, glycerin, gelatin or polyethylene glycols and molded into a suppository.

## Utility of the Present Invention

The compounds of Formula I are useful as protease inhibitors, particularly as inhibitors of cysteine and serine proteases, more particularly as inhibitors of cysteine proteases, even more particularly as inhibitors of cysteine proteases of the papain superfamily, yet more particularly as inhibitors of cysteine proteases of the cathepsin family, most particularly as inhibitors of cathepsin K. The present invention also provides useful compositions and formulations of said compounds, including pharmaceutical compositions and formulations of said compounds.

The present compounds are useful for treating diseases in which cysteine proteases are implicated, including infections by pneumocystis carinii, trypsanoma cruzi, trypsanoma brucei, and Crithidia fusiculata; as well as in schistosomiasis, malaria, tumor metastasis, metachromatic leukodystrophy, muscular dystrophy, amytrophy; and especially diseases in which cathepsin K is implicated, most particularly diseases of excessive bone or cartilage loss, including osteoporosis, gingival disease including gingivitis and periodontitis, arthritis, more specifically, osteoarthritis and rheumatoid arthritis, Paget's disease; hypercalcemia of malignancy, and metabolic bone disease.

Metastatic neoplastic cells also typically express high levels of proteolytic enzymes that degrade the surrounding matrix, and certain tumors and metastatic neoplasias may be effectively treated with the compounds of this invention.

For acute therapy, parenteral administration of a compound of Formula I is preferred. An intravenous infusion of the compound in 5% dextrose in water or normal saline, or a similar formulation with suitable excipients, is most effective, although an intramuscular bolus injection is also useful. Typically, the parenteral dose will be about 0.01 to about 100 mg/kg; preferably between 0.1 and 20 mg/kg, in a manner to maintain the concentration of drug in the plasma at a concentration effective to inhibit cathepsin K. The compounds are administered one to four times daily at a level to achieve a total daily dose of about 0.4 to about 400 mg/kg/day. The precise amount of an inventive compound which is therapeutically effective, and the route by which such compound is best administered, is readily determined by one of ordinary skill in the art by comparing the blood level of the agent to the concentration required to have a therapeutic effect.

The compounds of this invention may also be administered orally to the patient, in a manner such that the concentration of drug is sufficient to inhibit bone resorption or to achieve any other therapeutic indication as disclosed herein. Typically, a pharmaceutical composition containing the compound is administered at an oral dose of between about 0.1 to about 50 mg/kg in a manner consistent with the condition of the patient. Preferably the oral dose would be about 0.5 to about 20 mg/kg.

No unacceptable toxicological effects are expected when compounds of the present invention are administered.

**Biological Assays**

The compounds of this invention may be tested in one of several biological assays to determine the concentration of compound which is required to have a given pharmacological effect.

**Determination of cathepsin K proteolytic catalytic activity**

All assays for cathepsin K were carried out with human recombinant enzyme. Standard assay conditions for the determination of kinetic constants used a fluorogenic peptide substrate, typically Cbz-Phe-Arg-AMC, and were determined in 100 mM Na acetate at pH 5.5 containing 20 mM cysteine and 5 mM EDTA. Stock substrate solutions were prepared at concentrations of 10 or 20 mM in DMSO with 20 uM final substrate concentration in the assays. All assays contained 10% DMSO. Independent experiments found that this level of DMSO had no effect on enzyme activity or kinetic constants. All assays were conducted at ambient temperature. Product fluorescence (excitation at 360 nM; emission at 460 nM) was monitored with a Perceptive Biosystems Cytofluor II fluorescent plate reader. Product progress curves were generated over 20 to 30 minutes following formation of AMC product.

**Inhibition studies**

Potential inhibitors were evaluated using the progress curve method. Assays were carried out in the presence of variable concentrations of test compound. Reactions were initiated by addition of enzyme to buffered solutions of inhibitor and substrate. Data analysis was conducted according to one of two procedures depending on the appearance of the progress curves in the presence of inhibitors. For those compounds whose progress curves were linear, apparent inhibition constants $(K_{i,app})$ were calculated according to equation 1 (Brandt et al., Biochemitsry, 1989, 28, 140):

$$v = V_m A / [K_a(1 + I/K_{i,\,app}) + A] \qquad (1)$$

where $\nu$ is the velocity of the reaction with maximal velocity $V_m$ , $A$ is the concentration of substrate with Michaelis constant of $K_a$, and $I$ is the concentration of inhibitor.

For those compounds whose progress curves showed downward curvature characteristic of time-dependent inhibition, the data from individual sets was analyzed to give $k_{obs}$ according to equation 2:

$$[\mathrm{AMC}] = v_{ss}\, t + (v_0 - v_{ss})\, [1 - exp\, (-k_{obs} t)] / k_{obs} \qquad (2)$$

where [AMC] is the concentration of product formed over time $t$, $\nu_0$ is the initial reaction velocity and $\nu_{ss}$ is the final steady state rate. Values for $k_{obs}$ were then analyzed as a linear function of inhibitor concentration to generate an apparent second order rate constant ($k_{obs}$ / inhibitor concentration or $k_{obs}$ / [I]) describing the time-dependent inhibition. A complete

discussion of this kinetic treatment has been fully described (Morrison et al., Adv. Enzymol. Relat. Areas Mol. Biol., 1988, 61, 201).

**Human Osteoclast Resorption Assay**

Aliquots of osteoclastoma-derived cell suspensions were removed from liquid nitrogen storage, warmed rapidly at 37°C and washed x1 in RPMI-1640 medium by centrifugation (1000 rpm, 5 min at 4°C). The medium was aspirated and replaced with murine anti-HLA-DR antibody, diluted 1:3 in RPMI-1640 medium, and incubated for 30 min on ice The cell suspension was mixed frequently.

The cells were washed x2 with cold RPMI-1640 by centrifugation (1000 rpm, 5 min at 4°C) and then transferred to a sterile 15 mL centrifuge tube. The number of mononuclear cells were enumerated in an improved Neubauer counting chamber.

Sufficient magnetic beads (5 / mononuclear cell), coated with goat anti-mouse IgG, were removed from their stock bottle and placed into 5 mL of fresh medium (this washes away the toxic azide preservative). The medium was removed by immobilizing the beads on a magnet and is replaced with fresh medium.

The beads were mixed with the cells and the suspension was incubated for 30 min on ice. The suspension was mixed frequently. The bead-coated cells were immobilized on a magnet and the remaining cells (osteoclast-rich fraction) were decanted into a sterile 50 mL centrifuge tube. Fresh medium was added to the bead-coated cells to dislodge any trapped osteoclasts. This wash process was repeated x10. The bead-coated cells were discarded.

The osteoclasts were enumerated in a counting chamber, using a large-bore disposable plastic pasteur pipette to charge the chamber with the sample. The cells were pelleted by centrifugation and the density of osteoclasts adjusted to $1.5x10^4$/mL in EMEM medium, supplemented with 10% fetal calf serum and 1.7g/litre of sodium bicarbonate. 3 mL aliquots of the cell suspension ( per treatment) were decanted into 15 mL centrifuge tubes. These cells were pelleted by centrifugation. To each tube 3 mL of the appropriate treatment was added (diluted to 50 uM in the EMEM medium). Also included were appropriate vehicle controls, a positive control (87MEM1 diluted to 100 ug/mL) and an isotype control (IgG2a diluted to 100 ug/mL). The tubes were incubate at 37°C for 30 min.

0.5 mL aliquots of the cells were seeded onto sterile dentine slices in a 48-well plate and incubated at 37°C for 2 h. Each treatment was screened in quadruplicate. The slices were washed in six changes of warm PBS (10 mL / well in a 6-well plate) and then placed into fresh treatment or control and incubated at 37°C for 48 h. The slices were then washed in phosphate buffered saline and fixed in 2% glutaraldehyde (in 0.2M sodium cacodylate) for 5 min., following which they were washed in water and incubated in buffer for 5 min at 37°C. The slices were then washed in cold water and incubated in cold acetate buffer / fast red garnet for 5 min at 4°C. Excess buffer was aspirated, and the slices were air dried following a wash in water.

The TRAP positive osteoclasts were enumerated by bright-field microscopy and were then removed from the surface of the dentine by sonication. Pit volumes were determined using the Nikon/Lasertec ILM21W confocal microscope.

**General**

Nuclear magnetic resonance spectra were recorded at either 250 or 400 MHz using, respectively, a Bruker AM 250 or Bruker AC 400 spectrometer. $CDCl_3$ is deuteriochloroform, DMSO-$d_6$ is hexadeuteriodimethylsulfoxide, and $CD_3OD$ is tetradeuteriomethanol. Chemical shifts are reported in parts per million (d) downfield from the internal standard tetramethylsilane. Abbreviations for NMR data are as follows: s = singlet, d = doublet, t = triplet, q = quartet, m = multiplet, dd = doublet of doublets, dt = doublet of triplets, app = apparent, br = broad. J indicates the NMR coupling constant measured in Hertz. Continuous wave infrared (IR) spectra were recorded on a Perkin-Elmer 683 infrared spectrometer, and Fourier transform infrared (FTIR) spectra were recorded on a Nicolet Impact 400 D infrared spectrometer. IR and FTIR spectra were recorded in transmission mode, and band positions are reported in inverse wavenumbers ($cm^{-1}$). Mass spectra were taken on either VG 70 FE, PE Syx API III, or VG ZAB HF instruments, using fast atom bombardment (FAB) or electrospray (ES) ionization techniques. Elemental analyses were obtained using a Perkin-Elmer 240C elemental analyzer. Melting points were taken on a Thomas-Hoover melting point apparatus and are uncorrected. All temperatures are reported in degrees Celsius.

Analtech Silica Gel GF and E. Merck Silica Gel 60 F-254 thin layer plates were used for thin layer chromatography. Both flash and gravity chromatography were carried out on E. Merck Kieselgel 60 (230-400 mesh) silica gel.

Where indicated, certain of the materials were purchased from the Aldrich Chemical Co., Milwaukee, Wisconsin, Chemical Dynamics Corp., South Plainfield, New Jersey, and Advanced Chemtech, Louisville, Kentucky.

## Examples

In the following synthetic Examples, temperature is in degrees Centigrade (°C). Unless otherwise indicated, all of the starting materials were obtained from commercial sources. Without further elaboration, it is believed that one skilled in the art can, using the preceding description, utilize the present invention to its fullest extent. These (Demonstration) Examples are given to illustrate the invention. With respect to the scope of the invention reference is made to the claims for what is reserved to the inventors hereunder.

(Demonstration) Example 1

Preparation of {(S)-1-[1-((S)-2-Benzyloxycarbonylamino-4-methyl-pentanoyl)-3-oxo-azepan-4-ylcarbamoyl}carbamic acid benzyl ester

a.) Allyl-pent-4-enyl-carbamic acid *tert*-butyl ester

To a suspension ofNaH (3.05 g, 76.33 mmol of 60% NaH in oil; washed with hexanes) in DMF (30 mL) was added *tert*-butyl N-allylcarbamate (6.0 g, 38.2 mmol) in a dropwise fashion. The mixture was stirred at room temperature for approximately 10 minutes whereupon 5-bromo-1-pentene (6.78 mL, 57.24 mmol) was added in a dropwise fashion. The reaction was heated to 40°C for approximately 2 hours whereupon the reaction was partitioned between ethyl acetate and water. The organic layer was washed with water (2 x's), brine, dried ($MgSO_4$), filtered and concentrated to give 10 grams of the title compound as an oil: MS(EI) 226 ($M+H^+$).

b.) 2,3,4,7-Tetrahydro-azepine-1-carboxylic acid *tert*-butyl ester

To a solution of compound of (Demonstration) Example 1a (4.5 g) in benzene was added the 2,6-diisopropylphenylimidoneophylidene molybdenum bis(*t*-butoxide) (600 mg). The reaction was heated to reflux for 1.5 hours whereupon the reaction was concentrated *in vacuo.* Chromatography (50% $CH_2Cl_2$:hexanes) of the residue gave 3.92 g of the product:

c.) 8-Oxa-3-aza-bicyclo[5.1.0]octane-3-carboxylic acid *tert*-butyl ester

To a solution of the compound of (Demonstration) Example 1b (3.0g, 15.2 mmol) in $CH_2Cl_2$ was added m-CPBA (7.8 g, 45.6 mmol). The mixture was stirred overnight at room temperature whereupon it was partitioned between $CH_2Cl_2$ and staurated $K_2CO_3$. The organic layer was washed with sat. $NaHCO_3$, water, brine, dried ($MgSO_4$), filtered and concentrated to give 3.11g of the title compound as an oil: MS(EI) 214 ($M+H^+$).

d.) 4-Azido-3-hydroxy-azepane-1-carboxylic acid *tert*-butyl ester

To a solution of the epoxide from (Demonstration) Example 1c (3.92 g, 20 mmol) in methanol:water (180 mL of an 8:1 solution) was added $NH_4Cl$ (3.18g, 60 mmol) and sodium azide (3.9 g, 60 mmol). The reaction was heated to 40°C until complete consumption of the starting epoxide was observed by TLC analysis. The majority of the solvent was removed *in vacuo* and the remaining solution was diluted with ethyl acetate and washed with water, brine dried ($Na_2SO_4$), filtered and concentrated. Column chromatography (40% ethyl acetate:hexanes) of the residue provided 3.43 g of the title compound.

e.) 4-Amino-3-hydroxy-azepane-1-carboxylic acid *tert*-butyl ester

To a solution of the azido alcohol of (Demonstration) Example 1d (3.4 g) and 10% Pd/C (catalytic) in ethyl acetate:methanol (2:1 solution) was affixed a balloon of hydrogen. The reaction was stirred until complete consumption of the starting material was observed by TLC analysis. The reaction was filtered to remove the catalyst and the filtrate was concentrated *in vacuo.* Column chromatography of the residue (25% methanol:dichloromethane) provided 2.57 g of the title compound: MS(EI) 231 ($M+H^+$).

f.) 4-((S)-2-benzyloxycarbonylamino-4-methyl-pentanoylamino)-3-hydroxy-azepane-1-carboxylic acid *tert* butyl ester

To a solution of the amino alcohol of (Demonstration) Example 1e (160 mg, 0.70 mmol) in $CH_2Cl_2$ was added EDC (134 mg), HOBt (94 mg) and Cbz-leucine (185 mg). The reaction was maintained at room temperature until complete consumption of the starting material was observed by TLC analysis. The reaction was diluted with ethyl acetate and

washed with 1N HCl, sat. $K_2CO_3$, water, brine, dried ($MgSO_4$), filtered and concentrated. Column chromatography of the residue (3% methanol:dichloromethane) gave 200 mg of the title compound: MS(EI) 478 ($M+H^+$), 500 ($M+Na^+$).

g.) [(S)-1-(3-hydroxy-azepan-4-ylcarbamoyl)-3-methyl-butyl]-carbamic acid benzyl ester

A solution of the compound of (Demonstration) Example 1f (200 mg, 0.42 mmol) in methanol (5 mL) was added 4M HCl in dioxane (5 mL). The reaction was stirred at room temperature for approximately 2 hours whereupon the solvent was removed *in vacuo* to provide 168 mg of the title compound: MS(EI) 378 ($M+H^+$).

h.) {(S)-1-[4-((S)-2-Benzyloxycarbonylamino-4-methyl-pentanoylamino)-3-hydroxyazepane-1-carbonyl]-3-methyl-butyl} carbamic acid benzyl ester

To a solution of the amine salt of (Demonstration) Example 1g (168 mg, 0.42 mmol) in $CH_2Cl_2$ was added EDC (81 mg), HOBt (57 mg), triethylamine (0.09 mL) and Cbz-leucine (111 mg). The reaction was stirred until complete by TLC analaysis. Workup followed by column chromatography (5% $CH_3OH$:$CH_2Cl_2$) provided 159 mg of the title compound: MS(EI) 625 ($M+H^+$).

i.) {(S)-1-[4-((S)-2-Benzyloxycarbonylamino-4-methyl-pentanoylamino)-3-oxoazepane-1-carbonyl]-3-methyl-butyl}carbamic acid benzyl ester

To a solution of the alcohol of (Demonstration) Example 1h (130 mg, 0.21 mmol) in DMSO was added TEA (0.17 mL) and pyridine sulfur trioxide complex (97 mg, 0.62 mmol). The reaction was stirred at room temperature for approximately 2 hours whereupon it was partitioned between ethyl acetate and water. The organic layer was washed with brine, dried ($MgSO_4$), filtered and concentrated. Column chromatography of the residue (5% $CH_3OH$:$CH_2Cl_2$) provided 100 mg of the title compound as a mixture of diastereomers: $^1H$ NMR ($CDCl_3$): $\delta$ 1.0 ( m, 12H), 1.5-2.1 ( m, 8H), 2.2 (m, 4H), 3.0 (m, 1H), 3.5 (d, 1H). 3.6 (d , 1H), 4.01 (m, 1H), 4.5 ( m, 2H), 4.7 (m, 1H), 5.0 ( m, 5H), 7.3 (m, 10H): MS (EI) 623($M+H^+$), 645 ($M+Na^+$). Separation of the diastereomers by HPLC provided diastereomer 1:MS (EI) 623 ($M+H^+$), 645 ($M+Na^+$) and diastereomer 2: MS (ES) 623 ($M+H^+$), 645 ($M+Na^+$).

(Demonstration) Example 2

Preparation of Naphthylene-2-carboxylic acid[(S)-1-(1-benzyl-3-oxo-azepan-4-ylcarbamoyl)-3-methyl-butyl]amide

a.) Allyl-pent-4-enyl-carbamic acid benzyl ester

To a suspension ofNaH (1.83 g, 76.33 mmol of 90% NaH) in DMF was added benzyl allyl-carbamic acid benzyl ester (7.3 g, 38.2 mmol) in a dropwise fashion. The mixture was stirred at room temperature for approximately 10 minutes whereupon 5-bromo-1-pentene (6.78 mL, 57.24 mmol) was added in a dropwise fashion. The reaction was heated to 40°C for approximately 4 hours whereupon the reaction was partitioned between dichloromethane and water. The organic layer was washed with water (2x's), brine, dried ($MgSO_4$), filtered and concentrated. Column chromatography of the residue (10% ethyl acetate:hexanes) provided 10.3 grams of the title compound as an oil: MS(EI) 260 ($M+H^+$).

b.) 2,3,4,7-Tetrahydro-azepine-1-carboxylic acid benzyl ester

To a solution of compound of (Demonstration) Example 2a (50 g) in dichloromethane was added bis(tricyclohexylphosphine)benzylidine ruthenium (IV) dichloride (5.0 g). The reaction was heated to reflux until complete as determined by TLC analysis. The reaction was concentrated *in vacuo.* Column chromatography of the residue (50% dichloromethane:hexanes) gave 35 g of the title compound: MS(EI) 232 ($M+H^+$).

c.) 8-Oxa-3-aza-bicyclo[5.1.0]octane-3-carboxylic acid benzyl ester

Following the general procedure of (Demonstration) Example 1c except substituting the compound of (Demonstration) Example 2b the title compound was prepared: MS(EI) 248 ($M+H^+$), 270 ($M+Na^+$).

d.) 4-azido-3-hydroxy-azepane-1-carboxylic acid benzyl ester

To a solution of the epoxide from (Demonstration) Example 2c (2.0 g, 8.1 mmol) in methanol:water (8:1 solution) was added $NH_4Cl$ (1.29 g, 24.3 mmol) and sodium azide (1.58 g, 24.30 mmol). The reaction was heated to 40°C until

complete consumption of the starting epoxide was observed by TLC analysis. The majority of the solvent was removed *in vacuo* and the remaining solution was partitioned between ethyl acetate and pH 4 buffer. The organic layer was washed with sat. $NaHCO_3$, water, brine dried ($MgSO_4$), filtered and concentrated. Column chromatography (20% ethyl acetate:hexanes) of the residue provided 1.3 g of the title compound: MS(EI) 291 ($M+H^+$) plus 0.14 g of trans-4-hydroxy-3-azido-hexahydro-1H-azepine

e.) 4-amino-3-hydroxy-azepane-1-carboxylic acid benzyl ester

To a solution of the azido alcohol of (Demonstration) Example 2d (1.1g, 3.79 mmol) in methanol was added triethylamine (1.5 mL, 11.37 mmol) and 1,3-propanedithiol (1.1 mL, 11.37 mL). The reaction was stirred until complete consumption of the starting material was observed by TLC analysis whereupon the reaction was concentrated *in vacuo.* Column chromatography of the residue (20% methanol:dichloromethane) provided 0.72 g of the title compound: MS(EI) 265 ($M+H^+$).

f.) 4-((S)-2-*tert*-Butoxycarbonylamino-4-methyl-pentanoylamino)-3-hydroxy-azepan-1-carboxylic acid benzyl ester

To a solution of the amino alcohol of (Demonstration) Example 2e (720 mg, 2.72 mmol) in $CH_2Cl_2$ was added EDC (521 mg), HOBt (368 mg) and N-Boc-leucine (630 mg). The reaction was maintained at room temperature until complete consumption of the starting material was observed by TLC analysis. The reaction was diluted with ethyl acetate and washed with 1N HCl, sat. $K_2CO_3$, water, brine, dried ($MgSO_4$), filtered and concentrated. Column chromatography of the residue (3% methanol:dichloromethane) gave 1.0 g of the title compound: MS(EI) 478 ($M+H^+$).

g.) [(S)-1-(3-Hydroxy-azepan-4-ylcarbamoyl)-3-methyl-butyl]-carbamic acid *tert* butyl ester

To a solution of the compound of (Demonstration) Example 2f (1.0g) and 10% Pd/C (catalytic) in ethyl acetate: methanol (2:1 solution) was affixed a balloon of hydrogen. The reaction was stirred until complete consumption of the starting material was observed by TLC analysis. The reaction was filtered to remove the catalyst and the filtrate was concentrated *in vacuo* to provide 0.82 g of the title compound: MS(EI) 344 ($M+H^+$).

h.) [(S)-1-(1-Benzyl-3-Hydroxy-azepan-4-ylcarbamoyl)-3-methyl-butyl]-carbamic acid *tert* butyl ester

To a solution of the compound of (Demonstration) Example 2g (0.69 g, 2.01 mmol) in $CH_2Cl_2$ was added benzaldehyde (0.32 mL, 3.01 mmol) followed by sodium triacetoxyborohydride (0.85 g, 4.02 mmol). The reaction was stirred until complete as determined by TLC analysis whereupon several drops of water were added to the reaction to destroy the excess sodium triacetoxyborohydride. The mixture was diluted with ethyl acetate washed with sat. $NaHCO_3$, water, brine, dried ($Na_2SO_4$), filtered and concentrated. Column chromatography of the residue (5% methanol:dichloromethane) gave 800 mg of the title compound: MS(ES) 434 ($M+H^+$).

i.) (S)-2-Amino-4-methyl-pentanoic acid (1-benzyl-3-hydroxy-azepan-4-yl)-amide

To a solution of the compound of (Demonstration) Example 2h (800 mg) in methanol (15 mL) was added 4M HCl in dioxane (15 mL). The reaction was stirred at room temperature overnight whereupon it was concentrated *in vacuo* to give 800 mg of the title compound: MS(ES) 334 (M+H+).

j.) Naphthylene-2-carboxylic acid [(S)-1-(1-benzyl-3-hydroxy-azepan-4-ylcarbamoyl)-3-methyl-butyl]-amide

To a solution of the amine salt of (Demonstration) Example 2i (200 mg, 0.49 mmol) in $CH_2Cl_2$ was added triethylamine (0.17 mL, 1.22 mmol), EDC (103.5 mg, 0.54 mmol), HOBt (73 mg, 0.54 mmol) and 2-naphthoic acid (93 mg, 0.54 mmol). The reaction was stirred until complete by TLC analysis. The reaction was diluted with ethyl acetate and washed with sat. $NaHCO_3$, water, brine, dried ($Na_2SO_4$), filtered and concentrated. Column chromatography of the residue (5% methanol:dichloromethane) gave 0.14 g of the title compound: MS(EI) 488 ($M+H^+$).

k.) Naphthylene-2-carboxylic acid[(S)-1-(1-benzyl-3-oxo-azepan-4-ylcarbamoyl)-3-methyl-butyl]-amide

Following the general procedure of (Demonstration) Example 1i except substituting the compound of (Demonstration) Example 2j for the compound of (Demonstration) Example 1i the title compound was prepared: $^1H$ NMR ($EDCl_3$): δ 1.0 ( m, 6H), 1.5-2.1 ( m, 5H), 2.2 ( m, 2H), 2.9 (m, 1H), 3.2 (dd, 1H). 3.4 (m, 1H), 3.7 (m, 2H), 4.7 ( m, 1H), 5.2 ( m, 1H), 7.2-8.4 (m, 12H); MS(EI): 486 (M+H+, 100%). Separation of the diastereomers by HPLC provided diastereomer 1:

MS (EI) 486.3 (M+H+), and diastereomer 2: MS (ES) 486.3 (M+H+).

(Demonstration) Example 3

Preparation of Quinoline-2-carboxylic acid [(S)-1-(1-benzyl-3-oxo-azepan-4-ylcarbamoyl)-3-methyl-butyl]amide

a.) Quinoline-2-carboxylic acid [(S)-1-(1-benzyl-3-hydroxy-azepan-4-ylcarbamoyl)-3-methyl-butyl]amide

Following the general procedure of (Demonstration) Example 2j except substituting 2-quinolinecarboxylic acid for 2-naphthoic acid the title compound was prepared: MS(ES) 489 (M+H+).

b.) Quinoline-2-carboxylic acid [(S)-1-(1-benzyl-3-oxo-azepan-4-ylcarbamoyl)-3-methylbutyl]amide

Following the general procedure of (Demonstration) Example 1i except substituting the compound of (Demonstration) Example 3a the title compound was prepared: $^1H$ NMR ($CDCl_3$): δ 1.0 (m, 6H), 1.5-2.1 (m, 5H), 2.2 (m, 2H), 2.9 (m, 1H), 3.2 (dd, 1H). 3.4 (m, 1H), 3.7 (m, 2H), 4.7 (m, 1H), 5.2 (m, 1H), 7.2-8.4 (m, 11H); MS(EI): 487 (M+H+, 100%). The diastereomers were separated by preparative scale HPLC. Lyophilisation of the eluents provided diastereomer 1: MS (EI) 492.4 (M+H+), 983.7 2M+H+) and diastereomer 2: MS (EI) 492.4 (M+H+), 983.7 2M+H+).

(Demonstration) Example 4

Preparation of 4-{(S)-Methyl-2-[(Quinoline-2-carbonyl)-amino]pentanoylamino}-3-oxo-1-[2-(3-pyridin-2-yl-phenyl)-acetyl]azepanium

a.) 4-((S)-2-*tert*-Butoxycarbonylamino-4-methyl-pentanoylamino)-3-hydroxy-1-[2-(3-pyridin-2-yl-phenyl)-acetyl]-azepanium

To a solution of the compound of (Demonstration) Example 2g (0.5g, 1.46 mmol) in $CH_2Cl_2$ was added EDC (307 mg, 1.60 mmol), HOBt (216 mg, 1.60 mmol) and 3-(2-pyridyl)phenyl acetic acid (341 mg, 1.60 mmol). The reaction was stirred at room temperature until complete as determined by TLC analysis. Workup and column chromatography (2% methanol:dichloromethane) provided the title compound: MS(ES) 539 (M+H+).

b.) 4-((S)-Amino-4-methyl-pentanoylamino)-3-hydroxy-1-[2-(3-pyridin-2-yl-phenyl)-acetyl]-azepanium

To a solution of the compound of (Demonstration) Example 4a (1.3 g) dissolved in methanol (20 mL was added 4M HCl in dixoane (20 mL). The reaction was stirred until complete by TLC analysis whereupon it was concentrated *in vacuo* to give 1.1 g of the title compound: MS(EI) 439 (M+H+).

c.) 4-{(S)-Methyl-2-[(quinoline-2-carbonyl)-amino]pentanoylamino}-3-hydroxy-1-[2-(3-pyridin-2-yl-phenyl)-acetyl] azepanium

Following the procedure of (Demonstration) Example 3a except substituting the compound of (Demonstration) Example 4b the title compound was prepared: MS(EI) 594 (M+H+).

d.) 4-{(S)-Methyl-2-[(quinoline-2-carbonyl)-amino]pentanoylamino}-3-oxo-1-[2-(3-pyridin-2-yl-phenyl)-acetyl]azepanium

Following the procedure of (Demonstration) Example 1i except substituting the compound of (Demonstration) Example 4c the title compound was prepared: $^1H$ NMR ($CDCl_3$): δ 1.0 (m, 6H), 1.5-2.1 (m, 5H), 2.2 (m, 2H), 2.9 (m, 1H), 3.4 (dd, 1H). 3.8 (m, 3H), 4.1 (m, 2H), 4.7 (m, 3H), 5.4 (m, 1H), 7.2-8.4 (m, 14H); MS(EI): 592 (M+H+,100%).

(Demonstration) Example 5

Preparation of 3-Oxo-4-((S)-4-methyl-2-{[5-(2-morpholino-4-yl-ethoxy)-benzofuran-2-carbonyl]lamino}-pentanoylamino)-1-(4-methyl-pentanoyl)-azepanium

a.) 4-((S)-2-*tert*-butoxycarbonylamino-4-methyl-pentanoylamino)-3-hydroxy-1-(4-methyl-pentanoyl)-azepanium

Following the procedure of (Demonstration) Example 4a except substituting iso-caproic acid for 3-(2-pyridyl) phenyl acetic acid the title compound was prepared: MS(EI) 442 ($M+H^+$).

b.) 4-((S)-2-Amino4-methyl-pentanoylamino)-3-hydroxy-1-(4-methyl-pentanoyl)-azepanium

Following the procedure of (Demonstration) Example 2i except substituting the compound of (Demonstration) Example 5a the title compound was prepared: MS(EI) 342 ($M+H^+$).

c.) 3-Hydroxy-4-((S)-4-methyl-2-{[5-(2-morpholino-4-yl-ethoxy)-benzofuran-2-carbonyl]amino}-pentanoylamino)-1-(4-methyl-pentanoyl)-azepanium

To a solution of the compound of (Demonstration) Example 5b (200 mg, 0.53 mmol) in dichloromethane was added EDC (111 mg, 0.58 mmol), HOBt (78 mg, 0.58 mmol), TEA (0.11 mL, 0.79 mmol) and 5-(2-morpholin-4-yl-ethyloxy) benzofuran-2-carboxylic acid. The reaction was stirred at room temperature until complete as indicated by TLC analysis. Workup and column chromatography (5% methanol:dichloromethane) provided 160 mg of the title compound: MS(EI) 615 ($M+H^+$).

d.) 3-Oxo-4-((S)-4-methyl-2-{[5-(2-morpholino-4-yl-ethoxy)-benzofuran-2-carbonyl]amino}-pentanoylamino)-1-(4-methyl-pentanoyl)-azepanium

Following the procedure of (Demonstration) Example 1 i except substituting the compound of (Demonstration) Example 5d the title compound was prepared: $^1H$ NMR ($CDCl_3$): δ 1.0 (m, 12H), 1.5-2.1 (m, 8H), 2.2 (m, 2H), 2.3 (m, 1H), 2.4-2.5 (m, 2H), 2.6 (m 5H), 2.7 (m, 2H), 2.9 (m, 1H), 3.4 (m, 1H), 3.7 (m, 4H), 4.1 (m, 2H), 4.5-4.6 (m, 2H), 5.2 (m, 1H), 7.2-8.4 (m, 4H): MS(EI): 613 ($M+H^+$,100%). The diastereomers were separated by preparative scale HPLC. Lyophilisation of the eluents provided diastereomer 1 and diastereomer 2.

(Demonstration) Example 6

Preparation of 3-Oxo4-((S)-4-methyl-2-{[5-(2-morpholino-4-yl-ethoxy)-benzofuran-2-carbonyl]amino}-pentanoylamino)-1-benzenesulphonyl-azepanium

a.) 1-Benzenesulphonyl-4-((S)-2-*tert*-butoxycarbonylamino-methyl-pentanoylamino)-3-hydroxy-azepanium

To a solution of the amine of (Demonstration) Example 2g (0.5 g, 1.46 mmol) in dichloromethane was added triethylamine (0.4 mL, 2.92 mmol) followed by benzenesulphonyl chloride (0.28 mL, 2.18 mmol). The reaction was stirred at room temperature until complete as determined by TLC analysis. Workup and column chromatography (10% methanol: dichloromethane) provided 450 mg of the title compound: MS(EI) 484 ($M+H^+$).

b.) 4-((S)-2-Amino-methyl-pentanoylamino) 1-benzenesulphonyl-3-hydroxy-azepanium

Following the procedure of (Demonstration) Example 2i except substituting the compound of (Demonstration) Example 6a the title compound was prepared: MS(EI) 384 (M+H+).

c.) 3-Hydroxy-4-((S)-4-methyl-2-{[5-(2-morpholino-4-yl-ethoxy)-benzofuran-2-carbonyl]amino}-pentanoylamino)-1-benzenesulphonyl-azepanium

Following the procedure of (Demonstration) Example 5c except substituting the compound of (Demonstration) Example 6b the title compound was prepared: MS(EI) 657 (M+H+).

d.) 3-Oxo-4-((S)-4-methyl-2-{[5-(2-morpholino-4-yl-ethoxy)-benzofuran-2-carbonyl]amino}-pentanoylamino)-1-benzenesulphonyl-azepanium

Following the procedure of (Demonstration) Example 1 i except substituting the compound of (Demonstration) Example 6c the title compound was prepared: $^1H$ NMR ($CDCl_3$): δ 1.0 (m, 6H), 1.5-2.1 (m, 5H), 2.2 (m, 2H), 2.4 (m, 1H), 2.7 (m, 4H), 2.8 (m, 2H), 3.5 (m, 1H), 3.8 (m, 4H), 4.0 (m, 1H), 4.1 (m, 2H), 4.4 (m, 1H), 4.5 (m, 1H), 4.7 (m, 1H), 5.1 (m, 1H), 7.0 (m, 3H), 7.3 (m, 2H), 7.5 (m, 3H), 7.7 (m, 2H): MS(EI): 655 ($M+H^+$,100%).

Analysis of the diastereomeric mixture by analytical HPLC (40:60 to 45:55 $CH_3CN$:20 mm $KHPO_4$ (pH 7 buffer) 60 min. gradient 1 mL/min.; inertsil ODS-3 column 4.6 x 250 mm; UV detection at 215 nM) showed two peaks ($R_t$ = 44.6 mins. and 45.9 mins). The diastereomers were separated by preparative scale HPLC (40:60 to 50:50 $CH_3CN$: mm $KHPO_4$ (pH 7 buffer)gradient, 12 mL/min., 60 mins; inertsil ODS-3 column 250 x 20 mm; UV detection at 215 nM). Lyophilisation of the eluents provided diastereomer 1 (anal. $R_t$ = 44.6 mins.) and diastereomer 2 (anal. Rt = 45.9 mins).

(Demonstration) Example 7

Preparation of Benzofuran-2-carboxylic acid {(S)-3-methyl-1-[3-oxo-1-(pyridine-2-sulfonyl)-azepan-4-ylcarbamoyl]-butyl}amide

a.) (S)-2-Amino-4-methyl-pentanoic acid [3-hydroxy-1-(pyridine-2-sulfonyl)-azepan-4-yl]-amide

Following the procedure of (Demonstration) Examples 14a-b except substituting 2-pyridinesulfonyl chloride for benzenesulfonyl chloride of (Demonstration) Example 6a the title compound was prepared: MS(EI) 385 ($M+H^+$).

b.) Benzofuran-2-carboxylic acid {(S)-3-methyl-1-[3-hydroxy-1-(pyridine-2-sulfonyl)-azepan-4-ylcarbamoyl]-butyl} amide

To a solution of (S)-2-amino-4-methyl-pentanoic acid [3-hydroxy-1-(pyridine-2-sulfonyl)-azepan-4-yl]-amide of (Demonstration) Example 7a (0.15 g) in dichloromethane was added TEA (0.11 mL), HOBt (49 mg), EDC (69 mg) and benzofuran-2-carboxylic acid (58 mg). The reaction was stirred until complete. Workup and column chromatography (5% methanol:ethyl acetate) provided the title compound: MS(EI) 529 ($M+H^+$).

c.) Benzofuran-2-carboxylic acid {(S)-3-methyl-1-[3-oxo-1-(pyridine-2-sulfonyl)-azepan-4-ylcarbamoyl]-butyl}amide

Following the procedure of (Demonstration) Example 1 i except substituting the compound of (Demonstration) Example 7b the title compound was prepared: $^1H$ NMR ($CDCl_3$): δ 1.0 (m, 6H), 1.5-2.1 (m, 5H), 2.2 (m, 2H), 2.7 (m, 1H), 3.7 (dd, 1H). 4.0 (m, 1H), 4.7 (m, 2H), 5.0 (m, 1H), 7.2-7.3 (m, 3H), 7.4 (m, 4H), 7.6 (m, 1H), 8.0 (m, 2H), 8.7 (m, 1H); MS(EI): 527 ($M+H^+$, 40%).

The diastereomeric mixture was separated by HPLC to provide the faster eluting diastereoemer; $^1HNMR$: δ 1.0 (m, 6H), 1.5-2.1 (m, 5H), 2.2 (m, 2H), 2.7 (t, 1H), 3.7 (d, 1H); 4.0 (d, 1H), 4.7 (m, 2H), 5.0 (m, 1H), 7.2-7.3 (m, 3H), 7.4 (m, 4H), 7.6 (m, 1H), 8.0 (m, 2H), 8.7 (m, 1H); MS(EI): 527 ($M+H^+$, 100%), and the slower eluting diastereomer; $^1HNMR$: δ 1.0 (m, 6H), 1.5-2.1 (m, 5H), 2.2 (m, 2H), 2.7 (t, 1H), 3.7 (d, 1H); 4.0 (d, 1H), 4.7 (m, 2H), 5.0 (m, 1H), 7.2-7.3 (m, 3H), 7.4 (m, 4H), 7.6 (m, 1H), 8.0 (m, 2H), 8.7 (m, 1H); MS(EI): 527 ($M+H^+$, 100%).

(Demonstration) Example 8

Preparation of Benzo[b]thiophene-2-carboxylic acid {(S)-3-methyl-1-[3-oxo-1-[3-oxo-pyridine-2-sulfonyl)-azepan-4-ylcarbamoyl]-butyl}amide

a.) {(S)-1-[3-Hydroxy-1-(1-oxy-pyridine-sulfonyl)-azepan-4-ylcarbamoyl]-3-methylbutyl-carbamic acid *tert*-butyl ester

To a solution of [(S)-1-(3-hydroxy-azepan-4-ylcarbamoyl)-3-methyl-butyl]-carbamic acid *tert* butyl ester of (Demonstration) Example 2g (2.5 g) in dichloromethane (100 mL) and saturated sodium bicarbonate was added freshly prepared 2-pyidinesulphonyl chloride N-oxide (prepared by bubbling chlorine gas through a solution of 2-mercaptopyridine-N-oxidein 9M HCl for approximately 90 minutes. Removal of excess chlorine under vacuum provided the 2-pyridinesulfonyl chloride-N-oxide). The reaction was stirred at room temperature for 1 hour. Workup and column chromatography (10% methanol:dichloromethane) provided the title compound (2.0 g): MS(EI) 500 ($M+H^+$).

b.) (S)-2-Amino-4-methyl-pentanoic acid [3-hydroxy-1-(1-oxy-pyyridine-sulfonyl)-azepan-4-yl]-amide

To a solution of {(S)-1-[3-hydroxy-1-(1-oxy-pyridine-sulfonyl)-azepan-4-ylcarbamoyl]-3-methyl-butyl-carbamic acid *tert*-butyl ester of (Demonstration) Example 8a (2.0 g) in methanol (20 mL) was added 4M HCl in dioxane (20 mL). The reaction was stirred at room temperature for 1.5 hours whereupon it was concentrated to provide the title compound (1.8 g): MS(EI) 400 ($M+H^+$).

c.) Benzo[b]thiophene-2-carboxylic acid {(S)-3-methyl-10-[3-hydroxy-1-(1-oxypyridine-2-sulfonyl)-azepan-4-ylcarbamoyl]-butyl}amide

To a solution of (S)-2-amino-4-methyl-pentanoic acid [3-hydroxy-1-(1-oxy-pyyridine-sulfonyl)-azepan-4-yl]-amide of (Demonstration) Example 8b (0.25 g) in dichloromethane (12 mL) was added triethylamine (0.12 mL), EDC (0.11 g), HOBt (0.077 g) and benzo[b]thiophene-2-carboxylic acid. The reaction was stirred until complete. Workup and column chromatography (10% methanol: dichloromethane) provided the title compound (0.26 g): MS(EI) 560 ($M+H^+$).

d.) Benzo[b]thiophene-2-carboxylic acid {(S)-3-methyl-1-[3-oxo-1-(1-oxy-pyridine-2-sulfonyl)-azepan-4-ylcarbamoyl]-butyl}amide

Following the procedure of (Demonstration) Example 1 i except substituting benzo[b]thiophene-2-carboxylic acid {(S)-3-methyl-1-[3-hydroxy-1-(1-oxy-pyridine-2-sulfonyl)-azepan-4-ylcarbamoyl]-butyl}amide of (Demonstration) Example 8c the title compound was prepared: $^1H$ NMR ($CDCl_3$): δ 1.0 (m, 6H), 1.5-2.1 (m, 5H), 2.2 (m, 2H), 2.7 (m, 1H), 3.8 (q, 1H). 4.0 (m, 1H), 4.7 (m, 1H), 4.8 (m, 1H), 5.0 (m, 1H), 7.5 (m, 4H), 7.8 (m, 3H), 8.1-8.2 (m, 2H). MS(EI): 558 ($M^+$,100%) .

The diastereomeric mixture was separated by HPLC to provide the faster eluting diastereoemer; MS(EI): 558 ($M^+$,100%), and the slower eluting diastereomer; MS(EI): 558 ($M^+$,100%).

(Demonstration) Example 9

Preparation of Benzofuran-2-carboxylic acid {(S)-3-methyl-1-[(2,2',4-trideuterio)-3-oxo-1-(pyridine-2-sulfonyl)-azepan-4 ylcarbamoyl]-butyl}amide

To a solution of benzofuran-2-carboxylic acid {(S)-3-methyl-1-[3-oxo-1-(pyridine-2-sulfonyl)-azepan-4-ylcarbamoyl)-butyl}amide of (Demonstration) Example 7c (0.03 g) in $D_2O:CD_3OD$ (0.4:4 mL) was added triethylamine (0.04 mL). The reaction was heated to reflux for 2 hours whereupon it was concentrated and dried under vacuum. The residue was the redissolved in the same mixture and heated to reflux overnight. The reaction was concentrated and the residue purified by column chromatography (5% methanol:dichloromethane) to provide the title compound (0.02 g): $^1$HNMR: δ 1.0 (m, 6H), 1.5-2.2 (m, 6H), 2.7 (m, 1H), 4.1 (m, 1H), 4.7 (m, 2H), 7.4-8.0 (m, 8H), 8.7 (m, 1H); MS(EI): 529 ($M^+$, 45%). The diastereomeric mixture was separated by HPLC to provide the faster eluting diastereoemer: MS(EI): 530 ($M+H^+$, 100%) and the slower eluting diastereomer: MS(EI): 530 ($M+H^+$,100%).

Example 1

Preparation of Quinoline-2-carboxylic acid {(S)-3-methyl-1-[3-oxo-1-(pyridine-2-sulfonyl)-azepan-4-ylcarbamoyl]-butyl} amide

a.) Quinoline-2-carboxylic acid {(S)-3-methyl-1-[3-hydroxy-1-(pyridine-2-sulfonyl)-azepan-4-ylcarbamoyl]-butyl} amide

Following the procedure of (Demonstration) Example 7b except substituting quinoline-2-carboxylic acid for benzofuran-2-carboxylic acid the title compound was prepared: MS(EI) 540 ($M+H^+$).

b.) Quinoline-2-carboxylic acid {(S)-3-methyl-1-[3-oxo-1-(pyridine-2-sulfonyl)-azepan-4-ylcarbamoyl]-butyl}amide

Following the procedure of (Demonstration) Example 1 i except substituting the compound of (Demonstration) Example 1 a the title compound was prepared: $^1H$ NMR ($CDCl_3$): δ 1.0 (m, 6H), 1.5-2.1 (m, 5H), 2.2 (m, 2H), 2.7 (m, 1H), 3.7 (d, 1H). 4.1 (m, 1H), 4.7 (m, 2H), 5.0 (m, 1H), 7.0-7.2 (m, 1H), 7.3 (m, 1H), 7.5 (m, 1H), 7.7 (m, 1H), 7.8 (m, 3H), 8.1 (m, 1H), 8.3 (m, 2H), 8.7 (m, 2H); MS(EI): 538 ($M+H^+$,100%).

The diastereomeric mixture was separated by HPLC to provide the faster eluting diastereoemer; MS(EI): 538 ($M+H^+$, 100%), and the slower eluting diastereomer; MS(EI): 538 ($M+H^+$,100%).

Example 2

Preparation of 5-Methoxybenzofuran-2-carboxylic acid {(S)-3-methyl-1-[3-oxo-1-(pyridine-2-sulfonyl)-azepan-4-ylcarbamoyl]-butyl}amide

a.) 5-Methoxybenzofuran-2-carboxylic acid {(S)-3-methyl-1-[3-hydroxy-1-(pyridine-2-sulfonyl)-azepan-4-ylcarbamoyl]-butyl amide

Following the procedure of Example 28b except substituting 5-methoxybenzofuran-2-carboxylic acid for benzofuran-2-carboxylic acid the title compound was prepared: MS(EI) 559 ($M+H^+$).

b.) 5-Methoxybenzofuran-2-carboxylic acid {(S)-3-methyl-1-[3-oxo-1-(pyridine-2-sulfonyl)-azepan-4-ylcarbamoyl]-butyl} amide

Following the procedure of Example 1 i except substituting the compound of Example 59a the title compound was prepared: $^1H$ NMR ($CDCl_3$): δ 1.0 (m, 6H), 1.5-2.1 (m, 5H), 2.2 (m, 2H), 2.7 (m, 1H), 3.7 (d, 4H). 4.0 (m, 1H), 4.7 (m, 2H), 5.0 (m, 1H), 7.0 (m, 4H), 7.6 (m, 3H), 8.0 (m, 2H), 8.7 (m, 1H); MS(EI): 557 ($M+H^+$, 70%).

The diastereomeric mixture was separated by HPLC to provide the faster eluting diastereoemer; $^1H$NMR ($CDCl_3$): δ 1.0 (m, 6H), 1.5-2.1 (m, 5H), 2.2 (m, 2H), 2.7 (t, 1H), 3.7 (m, 4H). 4.0 (d, 1H), 4.7 (m, 2H), 5.0 (d, 1H), 7.0 (m, 4H), 7.6 (m, 3H), 8.0 (m, 2H), 8.7 (d, 1H); MS(EI): 557 ($M+H^+$,100%), and the slower eluting diastereomer; MS(EI): 557 ($M+H^+$,100%).

Example 3

Preparation of 5-Methoxybenzofuran-2-carboxylic acid {(S)-3-methyl-1-[3-oxo-1-(1-oxypyridine-2-sulfonyl)-azepan-4-ylcarbamoyl]-butyl}amide

a.) 5-Methoxybenzofuran-2-carboxylic acid {(S)-3-methyl-1-[3-hydroxy-1-(1-oxypyridine-2-sulfonyl)-azepan-4-ylcarbamoyl]-butyl}amide

Following the procedure of (Demonstration) Example 8c except substituting 5-methoxybenzofuran-2-carboxylic acid for benzo[b]thiophene-2-carboxylic acid the title compound was prepared: MS(EI) 574 ($M+H^+$).

b.) 5-Methoxybenzofuran-2-carboxylic acid {(S)-3-methyl-1-[3-oxo-1-(1-oxy-pyridine-2-sulfonyl)-azepan-4-ylcarbamoyl]-butyl}amide

Following the procedure of (Demonstration) Example 1 i except substuting 5-methoxybenzofuran-2-carboxylic acid {(S)-3-methyl-1-[3-hydroxy-1-(1-oxy-pyridine-2-sulfonyl)-azepan-4-ylcarbamoyl]-butyl}amide of (Demonstration) Example 3a the title compound was prepared: $^1H$ NMR ($CDCl_3$): δ 1.0 (m, 6H), 1.5-2.1 (m, 5H), 2.2 (m, 2H), 2.7 (m, 1H), 3.8 (m, 4H). 4.0 (m, 1H), 4,5 (t, 1H), 4.7 (m, 1H), 5.0 (m, 1H), 7.4-8.6 (m, 8H) 8.0-8.2 (m, 2H); MS(EI): 572 ($M^+$, 30%).

The diastereomeric mixture was separated by HPLC to provide the faster eluting diastereoemer; $^1H$NMR ($CDCl_3$): δ 1.0 (m, 6H), 1.5-2.1 (m, 5H), 2.2 (m, 2H), 2.7 (t, 1H), 3.7 (s, 3H), 3.8 (d, 1H). 4.0 (d, 1H), 4,7 (m, 1H), 4.8 (d, 1H), 5.0 (m, 1H), 7.4-8.6 (m, 8H) 8.0-8.2 (m, 2H); MS(EI): 573 ($M+H^+$,100%) and the slower eluting diastereomer; MS (EI): 573 ($M+H^+$,100%).

Example 4

Preparation of Thieno[3,2-b]thiophene-2-carboxylic acid {(S)-3-methyl-1-[3-oxo-1-(1-oxypyridine-2-sulfonyl)-azepan-4-ylcarbamoyl]-butyl}amide

a.) Thieno[3,2-b]thiophene-2-carboxylic acid {(S)-3-methyl-1-[3-hydroxy-1-(1-oxypyridine-2-sulfonyl)-azepan-4-ylcarbamoyl]-butyl}amide

Following the procedure of (Demonstration) Example 8c except substituting thieno[3,2-b]thiophene-2-carboxylic acid for benzo[b]thiophene-2-carboxylic acid the title compound was prepared: MS(EI) 566 ($M+H^+$).

b.) Thieno[3,2-b]thiophene-2-carboxylic acid {(S)-3-methyl-1-[3-oxo-1-(1-oxypyridine-2-sulfonyl)-azepan-4-ylcarbamoyl]-butyl}amide

Following the procedure of (Demonstration) Example 1 i except substuting thieno[3,2-b]thiophene-2-carboxylic acid {(S)-3-methyl-1-[3-hydroxy-1-(1-oxy-pyridine-2-sulfonyl)-azepan-4-ylcarbamoyl]-butyl}amide of (Demonstration) Example 4a the title compound was prepared: $^1$H NMR ($CDCl_3$): δ 1.0 (m,6H), 1.5-2.1 (m, 5H), 2.2 (m, 2H), 2.7 (m, 1H), 3.8 (q, 1H). 4.0 (m, 1H), 4,5 (t, 1H), 4.7 (m, 1H), 5.0 (m, 1H), 7.4-7.5 (m, 6H), 7.7 (d, 1H), 8.0-8.2 (m, 2H). MS(EI): 564 ($M^+$,100%).

The diastereomeric mixture was separated by HPLC to provide the faster eluting diastereoemer; $^1$HNMR ($CDCl_3$): δ 1.0 (m, 6H), 1.5-2.1 (m, 5H), 2.2 (m, 2H), 2.7 (t, 1H), 3.8 (d, 1H). 4.0 (d, 1H), 4,5 (m, 1H), 4.7 (d, 1H), 5.0 (m, 1H), 7.4-7.5 (m, 6H), 7.7 (d, 1H), 8.0-8.2 (m, 2H); MS(EI): 565 ($M+H^+$,100%) and the slower eluting diastereomer; MS (EI): 565 ($M+H^+$,100%).

Example 5

Preparation of Quinoxaline-2-carboxylic acid {(S)-3-methyl-1-[3-oxo-1-(1-oxy-pyridine-2-sulfonyl)-azepan-4-ylcarbamoyl]-butyl}amide

a.) Quinoxaline-2-carboxylic acid {(S)-3-methyl-1-[3-hydroxy-1-(1-oxy-pyridine-2-sulfonyl)-azepan-4-ylcarbamoyl]-butyl}amide

Following the procedure of (Demonstration) Example 8c except substituting quinoxaline-2-carboxylic acid for benzo[b]thiophene-2-carboxylic acid the title compound was prepared: MS(EI) 556 ($M+H^+$).

b.) Quinoxaline-2-carboxylic acid {(S)-3-methyl-1-[3-oxo-1-(1-oxy-pyridine-2-sulfonyl)-azepan-4-ylcarbamoyl]-butyl} amide

Following the procedure of (Demonstration) Example 1i except substuting quinoxaline-2-carboxylic acid {(S)-3-methyl-1-[3-hydroxy-1-(1-oxy-pyridine-2-sulfonyl)-azepan-4-ylcarbamoyl]-butyl}amide of (Demonstration) Example 5a the title compound was prepared: $^1$H NMR ($CDCl_3$): δ 1.0 (m, 6H), 1.5-2.1 (m, 5H), 2.2 (m, 2H), 2.7 (m, 1H), 3.8 (q, 1H). 4.0 (m, 1H), 4,5 (t, 1H), 4.7 (m, 1H), 5.0 (m, 1H), 7.4-7.5 (m, 2H), 7.9 (m, 1H), 8.0-8.4 (m, 4H, 9.6 (d, 1H); MS(EI): 554 ($M^+$, 100%).

The diastereomeric mixture was separated by HPLC to provide the faster eluting diastereoemer; MS(EI): 555 ($M+H^+$, 100%) and the slower eluting diastereomer; MS(EI): 555 ($M+H^+$, 100%).

Example 6

Preparation of 3-Methylbenzofuran-2-carboxylic acid {(S)-3-methyl-1-[3-oxo-1-(1-oxypyridine-2-sulfonyl)-azepan-4-ylcarbamoyl]-butyl}amide

a.) 3-Methylbenzofuran-2-carboxylic acid {(S)-3-methyl-1-[3-hydroxy-1-(1-oxypyridine-2-sulfonyl)-azepan-4-ylcarbamoyl]-butyl} amide

Following the procedure of (Demonstration) Example 8c except substituting 3-methylbenzofuran-2-carboxylic acid for benzo[b]tbiophene-2-carboxylic acid the title compound was prepared: MS(EI) 558 ($M^+$).

b.) 3-Methylbenzofuran-2-carboxylic acid {(S)-3-methyl-1-[3-oxo-1-(1-oxy-pyridine-2-sulfonyl)-azepan-4-ylcarbamoyl]-butyl}amide

Following the procedure of (Demonstration) Example 1i except substuting 3-methylbenzofuran-2-carboxylic acid {(S)-3-methyl-1-[3-hydroxy-1-(1-oxy-pyridine-2-sulfonyl)-azepan-4-ylcarbamoyl]-butyl}amide of (Demonstration) Example 6a the title compound was prepared: $^1$H NMR ($CDCl_3$): δ 1.0 (m, 6H), 1.5-2.1 (m, 5H), 2.2 (m, 2H), 2.5 (d, 3H), 2.7 (m, 1H), 3.8 (q, 1H); 4.0 (m, 1H), 4,5 (t, 1H), 4.7 (m, 1H), 5.0 (m, 1H), 7.4-8.0 (m, 6H), 8.1-8.2 (m, 2H); MS(EI): 556 ($M^+$, 100%).

The diastereomeric mixture was separated by HPLC to provide the faster eluting diastereoemer: $^1$H NMR ($CDCl_3$): δ 1.0 (m, 6H), 1.5-2.1 (m, 5H), 2.2 (m, 2H), 2.6 (s, 3H), 2.7 (t, 1H), 3.8 (d, 1H); 4.1 (d, 1H), 4,7 (m, 1H), 4.7 (d, 1H), 5.0 (m, 1H), 7.0 (m, 2H), 7.3 (m, 2H), 7.4 (m, 4H), 8.1 (d, 1H), 8.2 (d, 1H); MS(EI): 557 ($M+H^+$,100%) and the slower eluting diastereomer MS(EI): 557 ($M+H^+$,100%).

Example 7

Preparation of 3-Methylbenzofuran-2-carboxylic acid {(S)-3-methyl-1-[3 -oxo-1-(pyridine-2-sulfonyl)-azepan-4-ylcarbamoyl]-butyl}amide

a.) 3-Methylbenzofuran-2-carboxylic acid {(S)-3-methyl-1-[3-hydroxy-1-(pyridine-2-sulfonyl)-azepan-4-ylcarbamoyl]-butyl amide

Following the procedure of (Demonstration) Example 7b except substituting 3-methylbenzofuran-2-carboxylic acid for benzofuran-2-carboxylic acid the title compound was prepared: MS(EI) 542 ($M^+$).

b.) 3-Methylbenzofuran-2-carboxylic acid {(S)-3-methyl-1-[3-oxo-1-(pyridine-2-sulfonyl)-azepan-4-ylcarbamoyl]-butyl} amide

Following the procedure of (Demonstration) Example 1i except substituting 3-methylbenzofuran-2-carboxylic acid {(S)-3-methyl-1-[3-hydroxy-1-(pyridine-2-sulfonyl)-azepan-4-ylcarbamoyl]-butyl}amide of (Demonstration) Example 7a the title compound was prepared: $^1H$ NMR ($CDCl_3$): δ 1.0 (m, 6H), 1.5-2.2 (m, 6H), 2.2 (m, 2H), 2.6 (d, 3H), 2.7 (m, 1H), 3.8 (m, 1H), 4.1 (m, 1H), 4.7 (m, 2H), 5.2 (m, 1H), 7.4-8.0 (m, 7H); 8.7 (m, 1H); MS(EI): 540 ($M^+$, 100%).

The diastereomeric mixture was separated by HPLC to provide the faster eluting diastereoemer: $^1H$ NMR ($CDCl_3$): δ 1.0 (m, 6H), 1.5-2.2 (m, 6H), 2.2 (m, 2H), 2.6 (s, 3H), 2.7 (m, 1H), 3.8 (d, 1H); 4.1 (d, 1H), 4.7 (m, 2H), 5.2 (m, 1H), 7.4-8.0 (m, 7H); 8.7 (m, 1H); MS(EI): 541 ($M+H^+$,100%) and the slower eluting diastereomer MS(EI): 541 ($M+H^+$, 100%).

Example 8

Preparation of Thieno[3,2-b]thiophene-2-carboxylic acid {(S)-3-methyl-1-[3-oxo-1-(pyridine-2-sulfonyl)-azepan-4-ylcarbamoyl]-butyl}amide

a.) Thieno[3,2-b]thiophene-2-carboxylic acid {(S)-3-methyl-1-(3-hydroxy-1-(pyridine-2-sulfonyl)-azepan-4-ylcarbamoyl]-butyl}amide

Following the procedure of (Demonstration) Example 7b except substituting thieno[3,2-b]thiophene-2-carboxylic acid for benzofuran-2-carboxylic acid the title compound was prepared: MS(EI) 550 ($M^+$).

b.) Thieno[3,2-b]thiophene-2-carboxylic acid {(S)-3-methyl-1-[3-oxo-1-(pyridine-2 sulfonyl)-azepan-4-ylcarbamoyl]-butyl}amide

Following the procedure of (Demonstration) Example 1i except substituting thieno[3,2-b]thiophene-2-carboxylic acid {(S)-3-methyl-1-[3-hydroxy-1-(pyridine-2-sulfonyl)-azepan-4-ylcarbamoyl]-butyl}amide of (Demonstration) Example 8a the title compound was prepared: $^1H$ NMR ($CDCl_3$): δ 1.0 (m, 6H), 1.5-2.2 (m, 6H), 2.2 (m, 2H), 2.7 (m, 1H), 3.8 (m, 1H); 4.1 (m, 1H), 4.7 (m, 2H), 5.2 (m, 1H), 7.4-8.0 (m, 8H); 8.7 (m, 1H); MS(EI): 548 ($M^+$, 100%).

The diastereomeric mixture was separated by HPLC to provide the faster eluting diastereoemer: $^1HNMR$ ($CDCl_3$): δ 1.0 (m, 6H), 1.5-2.2 (m, 6H), 2.2 (m, 2H) 2.7 (t, 1H), 3.8 (d, 1H); 4.1 (d, 1H), 4.7 (m, 2H), 5.2 (m, 1H), 7.4-8.0 (m, 8H); 8.7 (d, 1H); MS(EI): 549 ($M+H^+$,100%) and the slower eluting diastereomer MS(EI): 549 ($M+H^+$, 100%).

The above specification and (Demonstration) Examples fully disclose how to make and use the compounds of the present invention. However, the present invention is not limited to the particular embodiments described hereinabove, but includes all modifications thereof within the scope of the following claims.

## Claims

**1.** A compound of formula I,

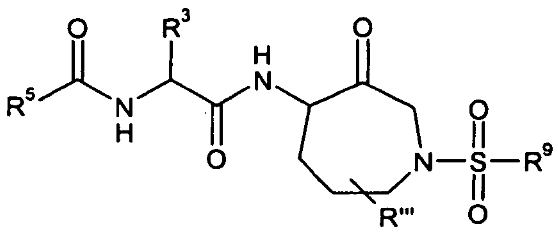

(I)

wherein,

$R^5$ is selected from the group consisting of 3-methyl-benzofuranyl-2-yl, thieno[3,2,6]thiophen-2-yl, 5-methoxy-benzofuran-2-yl, quinoxalin-2-yl and quinolin-2-yl;
R"' is hydrogen
$R^3$ is isobutyl;
R9 is selected from the group consisting of pyridine-2-yl and 1-oxy-pyridin-2-yl;

and pharmaceutical salts, hydrates and solvates thereof.

**2.** A compound according to claim 1, selected from the group consisting of:

quinoline-2-carboxylic acid {(S)-3-methyl-1-[3-oxo-1-(pyridine-2-sulfonyl)-azepan-4-ylcarbamoyl]-butyl}amide;
5-methoxy-benzofuran-2-carboxylic acid {(S)-3-methyl-1-[3-oxo-1-(pyridine-2-sulfonyl)-azepan-4-ylcarbamoyl]-butyl}amide;
5-methoxy-benzofuran-2-carboxylic acid {(S)-3-methyl-1-[3-oxo-1-(1-oxypyridine-2-sulfonyl)-azepan-4-ylcarbamoyl]-butyl}amide;
thieno[3,2-b]thiophene-2-carboxylic acid {(S)-3-methyl-1-[3-oxo-1-(1-oxypyridine-2-sulfonyl)-azepan-4-ylcarbamoyl]-butyl}amide;
quinoxaline-2-carboxylic acid {(S)-3-methyl-1-[3-oxo-1-(1-oxy-pyridine-2-sulfonyl)-azepan-4-ylcarbamoyl]-butyl}amide;
3-methyl-benzofuran-2-carboxylic acid {(S)-3-methyl-1-[3-oxo-1-(1-oxypyridine-2-sulfonyl)-azepan-4-ylcarbamoyl]-butyl} amide;
3-methyl-benzofuran-2-carboxylic acid {(S)-3-methyl-1-[3-oxo-1-(pyridine-2-sulfonyl)-azepan-4-ylcarbamoyl]-butyl}amide; and
thieno[3,2-b]thiophene-2-carboxylic acid {(S)-3-methyl-1-[3-oxo-1-(pyridine-2-sulfonyl)-azepan-4-ylcarbamoyl]-butyl}amide; or a pharmaceutical salt, hydrate or solvate thereof.

**3.** A pharmaceutical composition comprising a compound according to any one of claims 1 or 2 or a pharmaceutical salt, hydrate or solvate thereof and a pharmaceutically acceptable carrier, diluent or excipient.

**4.** A compound according to any one of claims 1 or 2 or a pharmaceutical salt, hydrate or solvate thereof, for use as an active therapeutic substance.

**5.** A compound according to claim 4 or a pharmaceutical salt, hydrate or solvate thereof, for use in inhibiting a protease selected from the group consisting of a cysteine protease and a serine protease.

**6.** A compound according to any one of claims 1 or 2 or a pharmaceutical salt, hydrate or solvate thereof, for use in the treatment of a disease **characterized by** bone loss.

**7.** A compound according to claim 6 wherein said disease is selected from rheumatoid arthritis, osteoporosis, periodontitis and gingivitis.

**8.** A compound according to any one of claims 1 or 2 or a pharmaceutical salt, hydrate or solvate thereof, for use in the treatment of a disease **characterized by** excessive cartilage or matrix degradation.

**9.** A compound according to claim 8, wherein said disease is osteoarthritis or rheumatoid arthritis.

**10.** A use of a compound according to any one of claims 1 or 2 or a pharmaceutical salt, hydrate or solvate thereof, for the manufacture of a medicament for use: in inhibiting a protease selected from the group consisting of a cysteine protease and a serine protease.

**11.** A use of a compound according to any one of claims 1 or 2 or a pharmaceutical salt, hydrate or solvate thereof, for the manufacture of a medicament: for use in the treatment of a disease **characterized by** bone loss or excessive cartilage or matrix degradation.

## Patentansprüche

**1.** Verbindung der Formel I,

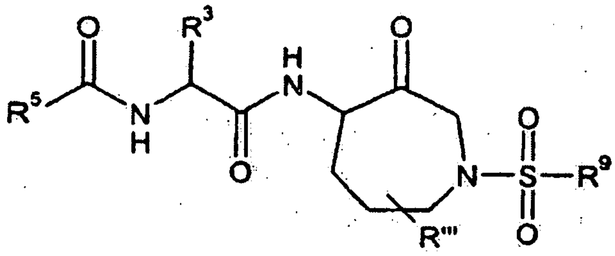

(I)

wobei

$R^5$ ausgewählt ist aus 3-Methylbenzofuranyl-2-yl, Thieno[3,2-b]thiophen-2-yl, 5-Methoxybenzofuran-2-yl, Chinoxalin-2-yl und Chinolin-2-yl;
R"' Wasserstoff ist,
$R^3$ Isobutyl ist;
$R^9$ ausgewählt ist aus Pyridin-2-yl und 1-Oxypyridin-2-yl;

und pharmazeutische Salze, Hydrate und Solvate davon.

**2.** Verbindung gemäß Anspruch 1, ausgewählt aus:

Chinolin-2-carbonsäure- {(S)-3-methyl-1-[3-oxo-1-(pyridin-2-sulfonyl)azepan-4-ylcarbamoyl]butyl}amid;
5-Methoxybenzofuran-2-carbonsäure-{(S)-3-methyl-1-[3-oxo-1-(pyridin-2-sulfonyl)azepan-4-ylcarbamoyl]butyl}amid;
5-Methoxybenzofuran-2-carbonsäure-{(S)-3-methyl-1-[3-oxo-1-(1-oxypyridin-2-sulfonyl)azepan-4-ylcarbamoyl]butyl}amid;
Thieno[3,2-b]thiophen-2-carbonsäure-{(S)-3-methyl-1-[3-oxo-1-(1-oxypyridin-2-sulfonyl)azepan-4-ylcarbamoyl]butyl}amid;
Chinoxalin-2-carbonsäure-{(S)-3-methyl-1-[3-oxo-1-(1-oxypyridin-2-sulfonyl)azepan-4-ylcarbamoyl]butyl}amid;
3-Methylbenzofuran-2-carbonsäure-{(S)-3-methyl-1-[3-oxo-1-(1-oxypyridin-2-sulfonyl)azepan-4-ylcarbamoyl]butyl}amid;
3-Methylbenzofuran-2-carbonsäure-{(S)-3-Methyl-1-[3-oxo-1-(pyridin-2-sulfonyl)azepan-4-ylcarbamoyl]butyl}amid; und
Thieno[3,2-b]thiophen-2-carbonsäure-{(S)-3-methyl-1-[3-oxo-1-(pyridin-2-sulfonyl)azepan-4-ylcarbamoyl]butyl}amid;

oder ein pharmazeutisches Salz, Hydrat oder Solvat davon.

**3.** Arzneimittel, umfassend eine Verbindung gemäß einem der Ansprüche 1 oder 2 oder ein pharmazeutisches Salz, Hydrat oder Solvat davon und ein(en) pharmazeutisch verträgliches(n) Träger, Verdünnungsmittel oder Exzpienten.

**4.** Verbindung gemäß einem der Ansprüche 1 oder 2 oder ein pharmazeutisches Salz, Hydrat oder Solvat davon, zur Verwendung als einen therapeutischen Wirkstoff.

**5.** Verbindung gemäß Anspruch 4 oder ein pharmazeutisches Salz, Hydrat oder Solvat davon, zur Verwendung bei der Hemmung einer Protease, ausgewählt aus einer Cysteinprotease und einer Serinprotease.

**6.** Verbindung gemäß einem der Ansprüche 1 oder 2 oder ein pharmazeutisches Salz, Hydrat oder Solvat davon, zur Verwendung bei der Behandlung einer durch Knochenverlust gekennzeichneten Erkrankung.

**7.** Verbindung gemäß Anspruch 6, wobei die Erkrankung ausgewählt ist aus rheumatoider Arthritis, Osteoporose, Periodontitis und Gingivitis.

**8.** Verbindung gemäß einem der Ansprüche 1 oder 2 oder ein pharmazeutisches Salz, Hydrat oder Solvat davon, zur Verwendung bei der Behandlung einer durch übermäßigen Knorpel- oder Matrixabbau gekennzeichneten Erkrankung.

**9.** Verbindung gemäß Anspruch 8, wobei die Erkrankung Osteoarthritis oder rheumatoide Arthritis ist.

**10.** Verwendung einer Verbindung gemäß einem der Ansprüche 1 oder 2 oder eines pharmazeutischen Salzes, Hydrats oder Solvats davon, zur Herstellung eines Medikaments zur Verwendung bei der Hemmung einer Protease, ausgewählt aus einer Cysteinprotease und einer Serinprotease.

**11.** Verwendung einer Verbindung gemäß einem der Ansprüche 1 oder 2 oder eines pharmazeutischen Salzes, Hydrats oder Solvats davon, zur Herstellung eines Medikaments zur Verwendung bei der Behandlung einer durch Knochnverlust oder durch übermäßigen Knorpel- oder Matrixabbau gekennzeichneten Erkrankung.

**Revendications**

**1.** Composé de formule I,

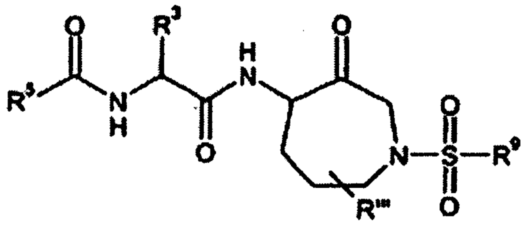

(I)

dans laquelle

$R^5$ est choisi dans le groupe consistant en des groupes 3-méthyl-benzofurannyl-2-yle, thiéno[3,2,*b*]-thiophène-2-yle, 5-méthoxybenzofuranne-2-yle, quinoxaline-2-yle et quinoléine-2-yle ;
R"' représente un atome d'hydrogène ;
$R^3$ représente un groupe isobutyle ;
$R^9$ est choisi dans le groupe consistant en des groupes pyridine-2-yle et 1-oxy-pyridine-2-yle ;

et des sels, hydrates et produits de solvatation pharmaceutiques.

**2.** Composé suivant la revendication 1, choisi dans le groupe consistant en :

{(S)-3-méthyl-1-[3-oxo-1-(pyridine-2-sulfonyl)-azépane-4-ylcarbamoyl]-butyl}amide d'acide quinoléine-2-carboxylique ;
{(S)-3-méthyl-1-[3-oxo-1-(pyridine-2-sulfonyl)-azépane-4-ylcarbamoyl]-butyl}amide d'acide 5-méthoxy-benzofuranne-2-carboxylique ;
{(S)-3-méthyl-1-[3-oxo-1-(1-oxy-pyridine-2-sulfonyl)-azépane-4-ylcarbamoyl]-butyl}amide d'acide 5-méthoxy-benzofuranne-2-carboxylique ;
{(S)-3-méthyl-1-[3-oxo-1-(1-oxy-pyridine-2-sulfonyl)-azépane-4-ylcarbamoyl]-butyl}amide d'acide thiéno[3,2-b]-thiophène-2-carboxylique ;
{(S)-3-méthyl-1-[3-oxo-1-(1-oxy-pyridine-2-sulfonyl)-azépane-4-ylcarbamoyl]-butyl}amide d'acide quinoxaline-2-carboxylique ;
{(S)-3-méthyl-1-[3-oxo-1-(1-oxy-pyridine-2-sulfonyl)-azépane-4-ylcarbamoyl]-butyl}amide d'acide 3-méthyl-benzofuranne-2-carboxylique ;
{(S)-3-méthyl-1-[3-oxo-1-(pyridine-2-sulfonyl)-azépane-4-ylcarbamoyl]-butyl}amide d'acide 3-méthyl-benzofuranne-2-carboxylique ; et
{(S)-3-méthyl-1-[3-oxo-1-(pyridine-2-sulfonyl)-azépane-4-ylcarbamoyl]-butyl}amide d'acide thiéno[3,2-b]thiophène-2-carboxylique ; ou un de ses sels, hydrates et produits de solvatation pharmaceutiques.

**3.** Composition pharmaceutique comprenant un composé suivant l'une quelconque des revendications 1 et 2, ou un de ses sels, hydrates et produits de solvatation pharmaceutiques et un support, diluant ou excipient pharmaceutiquement acceptable.

**4.** Composé suivant l'une quelconque des revendications 1 et 2 ou un de ses sels, hydrates et produits de solvatation pharmaceutiques, destiné à être utilisé comme substance thérapeutique active.

**5.** Composé suivant la revendication 4 ou un de ses sels, hydrates et produits de solvatation pharmaceutiques, destiné à être utilisé dans l'inhibition d'une protéase choisie dans le groupe consistant en une cystéine-protéase et une sérine-protéase.

**6.** Composé suivant l'une quelconque des revendications 1 et 2 ou un de ses sels, hydrates et produits de solvatation pharmaceutiques, destiné à être utilisé dans le traitement d'une maladie **caractérisée par** une perte de tissu osseux.

**7.** Composé suivant la revendication 6, dans lequel ladite maladie est choisie entre la polyarthrite rhumatoïde, l'ostéoporose, la périodontite et la gingivite.

**8.** Composé suivant l'une quelconque des revendications 1 et 2 ou un de ses sels, hydrates et produits de solvatation pharmaceutiques, destiné à être utilisé dans le traitement d'une maladie **caractérisée par** une dégradation excessive du cartilage ou de la matrice.

**9.** Composé suivant la revendication 8, dans lequel ladite maladie est l'arthrose ou la polyarthrite rhumatoïde.

**10.** Utilisation d'un composé suivant l'une quelconque des revendications 1 et 2 ou d'un de ses sels, hydrates et produits de solvatation pharmaceutiques, pour la production d'un médicament destiné à être utilisé dans l'inhibition d'une protéase choisie dans le groupe consistant en une cystéine-protéase et une sérine-protéase.

**11.** Utilisation d'un composé suivant l'une quelconque des revendications 1 et 2 ou d'un de ses sels, hydrates et produits de solvatation pharmaceutiques, pour la production d'un médicament destiné à être utilisé dans le traitement d'une maladie **caractérisée par** une perte de tissu osseux ou une dégradation excessive du cartilage ou de la matrice.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5501969 A
- WO 9404172 A
- EP 0603873 A1
- US 4518528 A
- EP 0525420 A1
- EP 0611756 A2
- US 9408868 W
- EP 0623592 A1
- GB 9101479 W
- US 4749792 A
- US 4638010 A
- US 9805336 B


### Non-patent literature cited in the description


- **BOSSARD, M. J. et al.** *J. Biol. Chem.,* 1996, vol. 271, 12517-12524
- **DRAKE, F.H. et al.** *J. Biol. Chem.,* 1996, vol. 271, 12511-12516
- **BROMME, D. et al.** *J. Biol. Chem.,* 1996, vol. 271, 2126-2132
- **POTEMPA, J. et al.** *Perspectives in Drug Discovery and Design,* 1994, vol. 2, 445-458
- **DELAISSE et al.** *Biochem. J.,* 1980, vol. 192, 365
- **DELAISSE et al.** *Biochem. Biophys. Res. Commun.,* 1984, vol. 125, 441
- **LERNER et al.** *J. Bone Min. Res.,* 1992, vol. 7, 433
- **DELAISSE et al.** *Bone,* 1987, vol. 8, 305
- **HILL et al.** *J. Cell. Biochem.,* 1994, vol. 56, 118
- **EVERTS et al.** *J. Cell. Physiol.,* 1992, vol. 150, 221
- **TEZUKA et al.** *J. Biol. Chem.,* 1994, vol. 269, 1106
- **INAOKA et al.** *Biochem. Biophys. Res. Commun.,* 1995, vol. 206, 89
- **SHI et al.** *FEBS Lett.,* 1995, vol. 357, 129
- **PALMER.** *J. Med. Chem.,* 1995, vol. 38, 3193
- **ELMORE et al.** *Biochem. J.,* 1968, vol. 107, 103
- **GARKER et al.** *Biochem. J.,* 1994, vol. 139, 555
- **GRAY et al.** *Tetrahedron,* 1977, vol. 33, 837
- **GUPTON et al.** *J. Biol. Chem.,* 1984, vol. 259, 4279
- **POWERS et al.** *J. Biol. Chem.,* 1984, vol. 259, 4288
- *J. Med. Chem.,* 1992, vol. 35, 4279
- **MCCONNELL et al.** *J. Med. Chem.,* vol. 33, 86
- **UMEZAWA et al.** *Meth. Enzymol.,* vol. 45 (678), E64
- **BARRETT.** *Biochem. J.,* vol. 201, 189
- **GRINDE.** *Biochem. Biophys. Acta,* vol. 701, 328
- *Eur. J. Biochem.,* 1984, vol. 158, 9
- COMPENDIUM OF ORGANIC SYNTHETIC METHODS. Wiley-Interscience, vol. I-VI
- **BODANSKY et al.** THE PRACTICE OF PEPTIDE SYNTHESIS. Springer-Verlag, 1984
- **E. GROSS ; J. MEIENHOFER.** *THE PEPTIDES,* 1979, vol. 1, 1-284
- **J.M. STEWART ; J.D. YOUNG.** SOLID PHASE PEPTIDE SYNTHESIS. Pierce Chemical Co, 1984
- **GREEN, T.W.** PROTECTIVE GROUPS IN ORGANIC SYNTHESIS. John Wiley & Sons, 1981
- **BRANDT et al.** *Biochemitsry,* 1989, vol. 28, 140
- **MORRISON et al.** *Adv. Enzymol. Relat. Areas Mol. Biol.,* 1988, vol. 61, 201